# EUROPEAN PATENT APPLICATION

(11) **EP 4 830 690 A1**
(43) Date of publication of application: **28.10.2026**
(21) Application number: 24907463.4
(22) Date of filing: 18.12.2024
(51) Int. Cl.: A61K 6/30, A61K 6/60, A61K 6/62, A61K 6/70

(54) **SINGLE-COMPONENT-TYPE DENTAL ADHESIVE COMPOSITION AND METHOD FOR PRODUCING SAME**

(30) Priority: 22.12.2023 JP 2023216792
(71) Applicant: Tokuyama Dental Corporation, Tokyo 110-0016 (JP)
(72) Inventor: NAKANISHI Kenta, Tokyo 110-0016 (JP); KISHI Hiroto, Tokyo 110-0016 (JP)
(74) Representative: Gill Jennings & Every LLP
(86) International application number: PCT/JP2024/044843
(87) International publication number: WO 2025/135079

(57) **Abstract**

Provided is a one-part dental adhesive composition, which has excellent adhesive strength, and in which the adhesive strength is unlikely to reduce even when a filling material is loaded more thickly. The one-part dental adhesive composition is used for bonding a photocurable filling material to a tooth structure, and includes polymerizable monomers (A) including acid monomers (A1), a photopolymerization initiator (B), water (C), a water-soluble organic solvent (D), and a monovalent to tetravalent metal compound (E). The acid monomers (A1) include a strong acid monomer (a1) and a carboxylic acid polymer-based polymerizable monomer (a2). The content of the monovalent to tetravalent metal compound (E) per 1 mol of the strong acid monomer (a1) is from 0.02 mol to 1 mol in terms of value converted into the number of moles of a metal ion for forming the monovalent to tetravalent metal compound (E). The content of the monovalent to tetravalent metal compound (E) per 1 g of the carboxylic acid polymer-based polymerizable monomer (a2) is from 0.0002 mol to 0.005 mol. The metal ion for forming the component (E) forms an aggregate with at least part of the strong acid monomer (a1).

## Description

### Technical Field

The present invention relates to a one-part dental adhesive composition and a method of producing the same.

### Background Art

A curable filling material called a composite resin has been used for the restoration of a tooth damaged by caries or the like. Such dental filling material loaded into a damaged portion of a tooth (hereinafter also simply referred to as "filling material") has almost no adhesiveness to a tooth structure for forming the tooth. Accordingly, the filling material is typically bonded to the tooth structure via an adhesive (dental adhesive composition). To obtain high adhesive strength, a general adhesive needs to be cured in advance. Here, the following three kinds of adhesives used in combination with a photocurable filling material that is cured only by light irradiation have been known from the viewpoint of (i) a curing mechanism: a photocurable type that is cured only by light irradiation; a chemical-curable type that is cured only by chemical polymerization; and a dual-curable type that is cured by photocuring and/or chemical curing. In addition, two kinds have been known from the viewpoint of (ii) the number of times of light irradiation required at the time of dental treatment: a single-light-irradiation type; and a double-light-irradiation type (e.g., Patent Literatures 1 to 4).

In the treatment of a tooth with an adhesive described in Patent Literature 1 (photocurable and double-light-irradiation type adhesive), the following Step 1(2) to Step 4(2) are sequentially performed, and post-treatment (e.g., the cutting or removal of an excess filling material after the curing) is further performed as required:
(Step 1(2)) the adhesive is applied to a damaged portion of a tooth and dried;
(Step 2(2)) light irradiation (first light irradiation) is performed on the adhesive after the drying to cure the adhesive;
(Step 3(2)) a photocurable filling material is loaded into the damaged portion of the tooth covered with the adhesive after the curing; and
(Step 4(2)) light irradiation (second light irradiation) is performed on the loaded filling material to cure the filling material.

In the double-light-irradiation type adhesive described in Patent Literature 1, light irradiation needs to be performed twice in an oral cavity during the treatment of a tooth. In addition, light irradiation for several seconds to several tens of seconds is required in Step 2(2) for curing the double-light-irradiation type adhesive described in Patent Literature 1. Accordingly, in the treatment of a tooth with the double-light-irradiation type adhesive described in Patent Literature 1, burden on a patient increases because of, for example, the lengthening of the treatment and heat generation along with the light irradiation. In addition, the double-light-irradiation type adhesive described in Patent Literature 1 may be contaminated by saliva, blood, or the like of the patient because the adhesive is exposed in his or her oral cavity at the time of the first light irradiation. Accordingly, there is required an adhesive that does not require light irradiation in a stage before the loading of the filling material. Adhesives (single-light-irradiation type adhesives) that do not require light irradiation in the stage before the loading of the filling material (process corresponding to Step 2(2) in the above-mentioned double-light-irradiation type adhesive) are, for example, adhesives listed in Patent Literatures 2 to 4.

The single-light-irradiation type adhesive described in Patent Literature 2 is a two-part adhesive (chemical-curable adhesive) configured so that chemical polymerization type curing may be advanced by mixing its first agent and second agent. However, the adhesive requires labor and time for mixing the first agent and the second agent during treatment.

The single-light-irradiation type adhesive described in Patent Literature 3 is the following dual-curable adhesive: after the loading of a filling material, photopolymerization type curing is advanced by light applied during the curing of the filling material; and chemical polymerization type curing is also advanced by the supply of an aromatic amine from the filling material. Accordingly, there is no need to spend labor or time for the curing treatment of the adhesive separately from the curing treatment of the filling material. However, the single-light-irradiation type adhesive described in Patent Literature 3 cannot be applied to any filling material other than an aromatic amine-containing filling material.

In addition, the single-light-irradiation type adhesive described in Patent Literature 4 is a photocurable adhesive that is cured only by light irradiation similarly to the double-light-irradiation type adhesive, but the adhesive is characterized by being blended with a highly active photopolymerization initiator. Accordingly, the adhesive described in Patent Literature 4 can be cured simultaneously with the curing of a filling material only by the light irradiation in Step 4(2) (i.e., single light irradiation) while Step 2(2) in the double-light-irradiation type adhesive is omitted. That is, in the treatment of a tooth with the single-light-irradiation type adhesive described in Patent Literature 4, the following Step 1(1) to Step 3(1) are sequentially performed, and post-treatment is further performed as required:
(Step 1(1)) the adhesive is applied to a damaged portion of a tooth and dried;
(Step 2(1)) a photocurable filling material is loaded into the damaged portion of the tooth covered with the adhesive after the drying; and
(Step 3(1)) light irradiation is performed on the loaded filling material and the adhesive covered with the filling material to cure the filling material and the adhesive.

The single-light-irradiation type adhesive (photocurable adhesive) described in Patent Literature 4 has a merit in that burden on a patient is small because the adhesive enables treatment quicker than that of the single-light-irradiation type adhesive (chemical polymerization type adhesive) described in Patent Literature 2. In addition, the single-light-irradiation type adhesive described in Patent Literature 4 also has a merit in that its versatility is high because there are no restrictions on the composition of the photocurable filling material utilized in combination with the adhesive, and hence the adhesive can be utilized in combination with photocurable filling materials of various compositions as compared to the single-light-irradiation type adhesive (dual-curable adhesive) described in Patent Literature 3.

### Citation List

### Patent Literature

[PTL 1] JP 2009-137960 A
[PTL 2] JP 2018-027913 A
[PTL 3] JP 2020-138911 A
[PTL 4] JP 2022-132064 A

### Summary of Invention

### Technical Problem

However, the single-light-irradiation/photocurable type adhesive described in Patent Literature 4 needs to be cured with irradiation light, which has reached an adhesive layer through a filling material layer, at the time of its use. Accordingly, the adhesive strength reduces when the thickness of the filling material layer arranged to cover the adhesive layer becomes larger. Accordingly, it tends to be difficult for the adhesive described in Patent Literature 4 to cope with a case requiring thicker loading of a filling material such as the treatment of a deep cavity.

The present invention has been made in view of the above-mentioned circumstances, and an object of the present invention is to provide: a single-light-irradiation/photocurable type one-part dental adhesive composition used in combination with a photocurable filling material, which has excellent adhesive strength, and in which the adhesive strength is unlikely to reduce even when the filling material is loaded more thickly; and a method of producing the same.

### Solution to Problem

The above-mentioned object is achieved by the present invention below. That is, a one-part dental adhesive composition of the present invention is used for bonding a photocurable filling material to a tooth structure, and includes: polymerizable monomers (A) including acidic group-containing polymerizable monomers (A1); a photopolymerization initiator (B); water (C); a water-soluble organic solvent (D); and a monovalent to tetravalent metal compound (E). The acidic group-containing polymerizable monomers (A1) include: a strong acidic group-containing polymerizable monomer (a1) having, in a molecule thereof, at least one radical polymerizable group and at least one strong acidic group selected from the group consisting of: a phosphono group {-P(=O)(OH)₂}; a dihydrogen phosphate monoester group {-O-P(=O)(OH)₂}; a hydrogen phosphate diester group {(-O-)₂P(=O)OH}; and a sulfo group (-SO₃H); and a carboxylic acid polymer-based polymerizable monomer (a2), which has, in a molecule thereof, at least one radical polymerizable group and at least one carboxyl group, which is free of the strong acidic group, and which has a weight average molecular weight (Mw) of from 2,000 to 120,000. A content of the monovalent to tetravalent metal compound (E) per 1 mol of the strong acidic group-containing polymerizable monomer (a1) is from 0.02 mol to 1 mol in terms of value converted into the number of moles of a metal ion for forming the monovalent to tetravalent metal compound (E). A content of the monovalent to tetravalent metal compound (E) per 1 g of the carboxylic acid polymer-based polymerizable monomer (a2) is from 0.0002 mol to 0.005 mol. The metal ion for forming the monovalent to tetravalent metal compound (E) forms an aggregate with at least part of the strong acidic group-containing polymerizable monomer (a1).

In one embodiment of the one-part dental adhesive composition of the present invention, the composition preferably includes 6 parts by mass to 60 parts by mass of the strong acidic group-containing polymerizable monomer (a1) per 100 parts by mass of the polymerizable monomers (A).

In another embodiment of the one-part dental adhesive composition of the present invention, the composition preferably includes 6 parts by mass to 40 parts by mass of the carboxylic acid polymer-based polymerizable monomer (a2) per 100 parts by mass of the polymerizable monomers (A).

In another embodiment of the one-part dental adhesive composition of the present invention, a weight average molecular weight in terms of standard polystyrene measured by the gel permeation chromatography analysis of the aggregate is preferably from 1,000 to 6,000.

In another embodiment of the one-part dental adhesive composition of the present invention, an acid value of the carboxylic acid polymer-based polymerizable monomer (a2) is preferably from 50 mg·KOH/g to 750 mg·KOH/g.

In another embodiment of the one-part dental adhesive composition of the present invention, the composition preferably includes 10 parts by mass to 500 parts by mass of the water-soluble organic solvent (D) per 100 parts by mass of the polymerizable monomers (A).

In another embodiment of the one-part dental adhesive composition of the present invention, the composition preferably further includes 5 parts by mass to 30 parts by mass of a filler (F) per 100 parts by mass of the polymerizable monomers (A).

In another embodiment of the one-part dental adhesive composition of the present invention, the monovalent to tetravalent metal compound (E) preferably includes at least one kind of metal selected from the group consisting of: Na(I); Mg(II); Ti(IV); Zr(IV); Zn(II); Ga(III); and Al(III).

A method of producing a one-part dental adhesive composition of the present invention includes at least: a raw material component preparation step of preparing at least a strong acidic group-containing polymerizable monomer (a1), a carboxylic acid polymer-based polymerizable monomer (a2), a photopolymerization initiator (B), water (C), a water-soluble organic solvent (D), and a monovalent to tetravalent metal compound (E) as raw material components; a first composition preparation step of preparing a first composition by selecting at least the strong acidic group-containing polymerizable monomer (a1) and the monovalent to tetravalent metal compound (E) after excluding, out of the raw material components prepared in the raw material component preparation step, the carboxylic acid polymer-based polymerizable monomer (a2) from options for mixing the raw material components with each other, and mixing the selected raw material components; and a second composition preparation step of preparing a second composition by selecting at least the carboxylic acid polymer-based polymerizable monomer (a2) from the raw material components, which are free from being selected in the first composition preparation step, and mixing the selected raw material component and the first composition, to produce the one-part dental adhesive composition of the present invention. Here, in the first composition preparation step, a mixing ratio between the strong acidic group-containing polymerizable monomer (a1) and a metal ion for forming the monovalent to tetravalent metal compound (E) is set to be 1 mol:from 0.02 mol to 1 mol. In the second composition preparation step, a mixing ratio between the carboxylic acid polymer-based polymerizable monomer (a2) and the monovalent to tetravalent metal compound (E) is set to be 1 g:from 0.0002 mol to 0.005 mol.

### Advantageous Effects of Invention

According to the present invention, there can be provided: the single-light-irradiation/photocurable type one-part dental adhesive composition used in combination with a photocurable filling material, which has excellent adhesive strength, and in which the adhesive strength is unlikely to reduce even when the filling material is loaded more thickly; and the method of producing the same.

### Description of Embodiments

A one-part dental adhesive composition and a method of producing the same according to embodiments of the present invention are described in detail below. In the following description, the numerical range of "from "x" to "y"" includes "x" and "y". That is, the expression "from "x" to "y"" means ""x" or more and "y" or less. 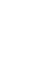 In addition, the term "(meth)acrylic" as used herein means both "acrylic" and "methacrylic". Similarly, the term "(meth)acrylate" means both "acrylate" and "methacrylate", and the term "(meth)acryloyl" means both "acryloyl" and "methacryloyl".

### 1. One-part Dental Adhesive Composition

The one-part dental adhesive composition of this embodiment is used for bonding a photocurable filling material to a tooth structure. From the viewpoints of the number of times of light irradiation required at the time of dental treatment and a curing mechanism, the composition is classified as a single-light-irradiation/photocurable type adhesive composition. In addition, the composition of the one-part dental adhesive composition of this embodiment satisfies at least the following requirements (i) to (v):
(i) the composition includes polymerizable monomers (A) including acidic group-containing polymerizable monomers (A1), a photopolymerization initiator (B), water (C), and a water-soluble organic solvent (D);
(ii) the composition further includes a monovalent to tetravalent metal compound (E);
(iii) the acidic group-containing polymerizable monomers (A1) include a strong acidic group-containing polymerizable monomer (a1) and a carboxylic acid polymer-based polymerizable monomer (a2);
(iv) the content of the monovalent to tetravalent metal compound (E) per 1 mol of the strong acidic group-containing polymerizable monomer (a1) is from 0.02 mol to 1 mol in terms of value converted into the number of moles of a metal ion for forming the monovalent to tetravalent metal compound (E), and the content of the monovalent to tetravalent metal compound (E) per 1 g of the carboxylic acid polymer-based polymerizable monomer (a2) is from 0.0002 mol to 0.005 mol; and
(v) the metal ion for forming the monovalent to tetravalent metal compound (E) forms an aggregate with at least part of the strong acidic group-containing polymerizable monomer (a1).

Here, the strong acidic group-containing polymerizable monomer (a1) is a compound having, in a molecule thereof, at least one radical polymerizable group and at least one strong acidic group selected from the group consisting of: a phosphono group {-P(=O)(OH)₂}; a dihydrogen phosphate monoester group {-O-P(=O)(OH)₂}; a hydrogen phosphate diester group {(-O-)₂P(=O)OH}; and a sulfo group (-SO₃H). In addition, the carboxylic acid polymer-based polymerizable monomer (a2) (also referred to as "carboxyl group-containing high-molecular weight polymerizable monomer") is a compound, which has, in a molecule thereof, at least one radical polymerizable group and at least one carboxyl group, which is free of the strong acidic group, and which has a weight average molecular weight (Mw) of from 2,000 to 120,000.

The single-light-irradiation/photocurable type one-part dental adhesive composition of this embodiment used in combination with a photocurable filling material during dental treatment has the composition described in the above-mentioned items (i) to (v). Accordingly, the composition can exhibit such effects that the composition has excellent adhesive strength and the adhesive strength is unlikely to reduce even when the filling material is loaded more thickly. Before finding the one-part dental adhesive composition of this embodiment exhibiting such effects, the inventors of the present invention have first examined the following problem with the related-art single-light-irradiation/photocurable type one-part dental adhesive composition listed in Patent Literature 4: its adhesive strength reduces as a filling material layer becomes thicker.

First, in the related-art single-light-irradiation/photocurable type one-part dental adhesive composition listed in Patent Literature 4 having the composition described in the above-mentioned item (i), to obtain high adhesive strength, a radical needs to be generated in a sufficient amount at the time of light irradiation in an adhesive layer in an uncured state formed by the application and drying (solvent removal) of the adhesive composition. This is because in this case, a high-strength adhesive layer can be formed by sufficiently curing the polymerizable monomer. However, when photopolymerization is advanced in the adhesive layer in the uncured state by utilizing irradiation light that has passed through the filling material layer in Step 3(1), as the thickness of the filling material layer increases, radical generation by the photopolymerization initiator becomes insufficient on an inner side (i.e., the adhesive layer in the uncured state) where the irradiation light is difficult to reach. Accordingly, when the thickness of the filling material layer is large, the strength of the adhesive layer after its curing reduces, and the adhesive layer after the curing having low strength is damaged by a stress along with the polymerization shrinkage of the filling material layer. Thus, the adhesive strength reduces.

To solve such problem, the inventors of the present invention have examined the blending of components (a carboxylic acid polymer and a metal ion) capable of forming an ionomer structure into the one-part dental adhesive composition. This is because even when the thickness of the filling material layer is large, relatively high adhesive strength may be easily secured at the end of Step 3(1) as long as an adhesive layer having an ionomer structure with high strength can be formed by a reaction between the carboxylic acid polymer and the metal ion included in the one-part dental adhesive composition at the time of the drying (at the time of the solvent removal) in Step 1(1). However, when the carboxylic acid polymer and the metal ion coexist in a solution, the following problem typically occurs: the ionomer formation reaction proceeds rapidly, and a reaction product formed by the reaction precipitates. Accordingly, the inventors of the present invention have conceived that the composition of the one-part dental adhesive composition including the carboxylic acid polymer and the metal ion needs to be designed so that the following may be achieved: while the ionomer formation reaction is suppressed as much as possible in the one-part dental adhesive composition, the ionomer formation reaction proceeds significantly when the solvent is removed from the one-part dental adhesive composition. In view of the foregoing, the inventors have conducted further investigations.

As a result, the inventors of the present invention have found the one-part dental adhesive composition of this embodiment having the composition described in the above-mentioned items (i) to (v). In addition, the one-part dental adhesive composition of this embodiment has features described in the following items (1) to (3):
(1) the metal ion existing in the form of the monovalent to tetravalent metal compound (E) in the one-part dental adhesive composition associates with the strong acidic group-containing polymerizable monomer (a1) to form an aggregate soluble in the water-soluble organic solvent (D);
(2) the aggregate and a carboxylic acid polymer can stably coexist without reacting with each other in the one-part dental adhesive composition in a state before the volatilization of its solvent components (the water (C) and the organic solvent (D)), or in other words, in a state of sufficiently containing the solvent components (consequently, the one-part dental adhesive composition has excellent storage stability); and
(3) in the case where the carboxylic acid polymer-based polymerizable monomer (a2) is used as the carboxylic acid polymer, an adhesive layer including a firm ionomer structure having relatively high strength is formed when the solvent components are volatilized and removed from the one-part dental adhesive composition in Step 1(1).

Here, when dental treatment is performed with the one-part dental adhesive composition of this embodiment having the features described in the above-mentioned items (1) to (3), the following Step 1(1) to Step 3(1) are sequentially performed, and post-treatment is further performed as required, similarly to the single-light-irradiation type adhesive (photocurable adhesive) described in Patent Literature 4:
(Step 1(1)) the adhesive is applied to a damaged portion of a tooth and dried;
(Step 2(1)) a photocurable filling material is loaded into the damaged portion of the tooth covered with the adhesive after the drying; and
(Step 3(1)) light irradiation is performed on the loaded filling material and the adhesive covered with the filling material to cure the filling material and the adhesive.

In Step 3(1), when the light irradiation is performed, the filling material in an uncured state formed in Step 2(1) is photocured, and at the same time, the adhesive layer in the uncured state formed in Step 1(1) is also photocured. Here, the degree of the photocuring of the adhesive layer in the uncured state formed in Step 1(1) reduces as the thickness of the filling material layer in the uncured state increases. This is because the intensity of the irradiation light reaching the uncured adhesive layer through the filling material layer in the uncured state at the time of the light irradiation reduces in proportion to the thickness of the filling material layer in the uncured state. In addition, the foregoing is a phenomenon common to the single-light-irradiation type adhesive (photocurable adhesive) described in Patent Literature 4 and the one-part dental adhesive composition of this embodiment.

However, in contrast to the single-light-irradiation type adhesive (photocurable adhesive) described in Patent Literature 4, in the one-part dental adhesive composition of this embodiment, the ionomer structure is further formed in the adhesive layer in the uncured state at the time of the completion of Step 1(1). Accordingly, even when the thickness of the filling material layer in the uncured state increases (in other words, even when <i> the photocuring of the adhesive layer becomes insufficient owing to a reduction in intensity of the irradiation light reaching the adhesive layer in the uncured state, and <ii> a peeling stress between the photocured adhesive layer and the damaged portion of the tooth increases owing to an increase in amount of the polymerization shrinkage of the filling material layer at the time of the photocuring), the adhesive layer after the photocuring can secure strength required to suppress its peeling from the damaged portion of the tooth by virtue of the presence of the ionomer structure. Accordingly, even in the case where the thickness of the filling material layer is large, a reduction in adhesive strength can be consequently suppressed in a significant manner as compared to that in the case where the thickness of the filling material layer is small.

Next, details of the respective components for forming the one-part dental adhesive composition of this embodiment are described.

### 1-1. Polymerizable Monomers (A)

The polymerizable monomers (A) used in the one-part dental adhesive composition of this embodiment include at least the acidic group-containing polymerizable monomers (A1). In addition, at least the strong acidic group-containing polymerizable monomer (a1) and the carboxylic acid polymer-based polymerizable monomer (a2) are used as the acidic group-containing polymerizable monomers (A1). Any other acidic group-containing polymerizable monomer (a3) may be further used as the acidic group-containing polymerizable monomers (A1) as required. An acidic group-free polymerizable monomer (A2) may also be used as the polymerizable monomers (A) as required.

### 1-1-1. Acidic Group-containing Polymerizable Monomers (A1)

### <<Strong Acidic Group-containing Polymerizable Monomer (Strong Acid Monomer) (a1)>>

The strong acidic group-containing polymerizable monomer (strong acid monomer) (a1) is a polymerizable monomer including, in a molecule thereof, at least one radical polymerizable group and at least one strong acidic group. The strong acid monomer (a1) mainly improves the adhesiveness to each of: a tooth structure (dentin or enamel); a base metal (iron, nickel, chromium, cobalt, tin, aluminum, copper, titanium, or the like, or an alloy including such metal as a main component); and a metal oxide including a metal such as zirconium and oxygen as main components (a zirconia ceramic, alumina, titania, or the like). In addition, the strong acid monomer (a1) makes the adhesiveness of a filling material to the tooth structure satisfactory because the monomer has a demineralizing effect on the tooth structure and a high improving effect on permeability into the tooth structure. Accordingly, in the one-part dental adhesive composition of this embodiment, excellent adhesive strength can be secured by using the strong acid monomer (a1).

In the one-part dental adhesive composition of this embodiment, the strong acid monomer (a1) associates with a metal ion generated by the dissociation of the monovalent to tetravalent metal compound (E) to form an aggregate. The formation of the aggregate in the one-part dental adhesive composition can prevent the insolubilization due to the formation of an ionomer even when the metal ion generated by the dissociation from the monovalent to tetravalent metal compound (E) and the carboxylic acid polymer-based polymerizable monomer (a2) are stored in a coexisting state in one part.

The strong acid monomer (a1) has an acryloyl group, a methacryloyl group, an acrylamide group, a methacrylamide group, a styryl group, or the like as a radical polymerizable group. The radical polymerizable group is preferably an acryloyl group and/or a methacryloyl group out of those groups from the viewpoint of adhesiveness to the filling material or the like.

In addition, the strong acid monomer (a1) needs to have a strong acidic group having a pKa smaller than that of the carboxylic acid polymer-based polymerizable monomer (a2). When a monomer having an acidic group having a large pKa such as a carboxyl group is used instead of the strong acid monomer (a1), a difference in acidity between the monomer and the carboxylic acid polymer-based polymerizable monomer (a2) becomes smaller. As a result, the carboxylic acid polymer-based polymerizable monomer (a2) aggregate via a metal ion to form an insoluble ionomer during its storage in one part. Accordingly, when the adhesive composition is applied and dried, an ionomer structure cannot be formed in the adhesive layer. Thus, the strong acid monomer (a1) includes at least one strong acidic group selected from a phosophono group {-P(=O)(OH)₂}, a dihydrogen phosphate monoester group {-O-P(=O)(OH)₂}, a hydrogen phosphate diester group {(-O-)₂P(=O)OH}, and a sulfo group (-SO₃H). The strong acid monomer (a1) more preferably includes at least one kind of strong acidic group selected from a dihydrogen phosphate monoester group and a hydrogen phosphate diester group because the monomer has so high stability in water that the dissolution of a smear layer on a tooth surface or tooth demineralization can be performed moderately.

Examples of the strong acid monomer (a1) that may be suitably used may include the following monomers. That is, examples of the strong acid monomer (a1) having a sulfo group may include 2-methacrylamido-2-methylpropanesulfonic acid, 2-acrylamido-2-methylpropanesulfonic acid, p-vinylbenzene sulfonic acid, and vinyl sulfonic acid.

Examples of the strong acid monomer (a1) having a dihydrogen phosphate monoester group or a hydrogen phosphate diester group may include 2-(meth)acryloyloxyethyl dihydrogen phosphate, bis[2-(meth)acryloyloxyethyl] hydrogen phosphate, 2-(meth)acryloyloxyethyl phenyl hydrogen phosphate, 6-(meth)acryloyloxyhexyl dihydrogen phosphate, 6-(meth)acryloyloxyhexyl phenyl hydrogen phosphate, 10-(meth)acryloyloxydecyl dihydrogen phosphate, 1,3-di(meth)acryloylpropane-2-dihydrogen phosphate, 1,3-di(meth)acryloylpropane-2-phenyl hydrogen phosphate, bis[5-{2-(meth)acryloyloxyethoxycarbonyl}heptyl] hydrogen phosphate, 11-(meth)acryloyloxy tetrapropylene glycol hydrogen phosphate, and 14-(meth)acryloyloxy pentapropylene glycol hydrogen phosphate.

Examples of the strong acid monomer (a1) having a phosophono group may include vinyl phosphonic acid and p-vinyl benzene phosphonic acid.

In particular, from the viewpoint of the adhesiveness to each of a tooth structure and an oxide ceramic (zirconia), the strong acid monomer (a1) is preferably 10-(meth)acryloyloxydecyl dihydrogen phosphate. The strong acid monomers (a1) may be used alone or in combination thereof.

In addition, from the viewpoint of achieving both the adhesiveness to a tooth structure and the strength of the adhesive layer after its curing, the blending amount of the strong acid monomer (a1) is preferably from 3 parts by mass to 60 parts by mass, more preferably from 6 parts by mass to 60 parts by mass, still more preferably from 8.5 parts by mass to 50 parts by mass per 100 parts by mass of the polymerizable monomers (A) included in the one-part dental adhesive composition of this embodiment.

### <<Carboxylic Acid Polymer-based Polymerizable Monomer (a2)>>

The carboxylic acid polymer-based polymerizable monomer (a2) is a polymerizable monomer, which includes, in a molecule thereof, at least one radical polymerizable group and at least one carboxyl group, which is free of a strong acidic group, and which has a weight average molecular weight of from 2,000 to 120,000. The term "strong acidic group" as used herein means a strong acidic group for forming a molecule of the strong acid monomer (a1). When the solvent components are volatilized and removed from the one-part dental adhesive composition of this embodiment, the carboxylic acid polymer-based polymerizable monomer (a2) contributes to an improvement in strength of the adhesive layer by forming an ionomer with the metal ion for forming the monovalent to tetravalent metal compound (E). In addition, the carboxylic acid polymer-based polymerizable monomer (a2) improves the adhesiveness to: a tooth structure (dentin or enamel); or a base metal (iron, nickel, chromium, cobalt, tin, aluminum, copper, titanium, or the like, or an alloy including such metal as a main component).

The carboxylic acid polymer-based polymerizable monomer (a2) has an acryloyl group, a methacryloyl group, an acrylamide group, a methacrylamide group, a styryl group, or the like as a radical polymerizable group. The radical polymerizable group is preferably an acryloyl group and/or a methacryloyl group out of those groups from the viewpoint of adhesiveness to a filling material or the like. When the carboxylic acid polymer-based polymerizable monomer (a2) does not include a radical polymerizable group, the monomer cannot copolymerize with any polymerizable monomer other than the carboxylic acid polymer-based polymerizable monomer (a2) existing in the adhesive layer in the uncured state, and hence the adhesive strength reduces. A polyacrylic chain or a polymethacrylic chain is preferred as a main chain for forming a molecule of the carboxylic acid polymer-based polymerizable monomer (a2) from the viewpoints of stability and biocompatibility.

The carboxylic acid polymer-based polymerizable monomer (a2) preferably includes a first repeating unit described in the following item (i) and a second repeating unit described in the following item (ii):
(i) a first repeating unit in which a functional group bonded to a carbon atom for forming its main chain selected from the group consisting of: an ethylene chain; and a propylene chain is a carboxyl group; and
(ii) a second repeating unit including a linking group, which is bonded to a carbon atom for forming its main chain selected from the group consisting of: an ethylene chain; and a propylene chain, and has an acryloyl group, a methacryloyl group, an acrylamide group, or a methacrylamide group at a terminal thereof. Here, the number of atoms for forming the main chain of the linking group (provided that when the main chain includes a cyclic structure in addition to a linear structure, the number of atoms is counted as the number of atoms included in a path including a smaller number of atoms out of two paths connecting a first atom and a second atom for forming nodes between the linear structure and the cyclic structure) is preferably from 3 to 30. In addition, when the main chain is formed of an ethylene chain, a carboxyl group bonded to a carbon atom of the ethylene main chain may be included.

A compound represented by the following general formula (1), (2), or (3) is preferred as the carboxylic acid polymer-based polymerizable monomer (a2).

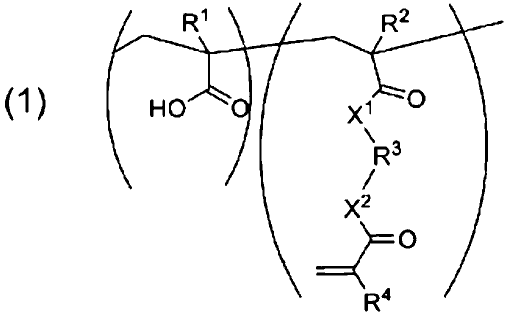

In the general formula (1), R¹ and R² each represent a hydrogen atom, a methyl group, or a carboxyl group, R³ represents an alkylene group having 1 to 15 carbon atoms or a polyethylene oxide segment having 2 to 7 repeating units, and R⁴ represents a hydrogen atom or a methyl group. In addition, X¹ and X² each represent -O- or -NH-. The number of repetitions "a" of a repeating unit on the left side and the number of repetitions "b" of a repeating unit on the right side in the general formula (1) may be identical to or different from each other. In addition, a ratio (a:b) between both the repeating units is preferably from 95:5 to 5:95, more preferably from 80:20 to 20:80.

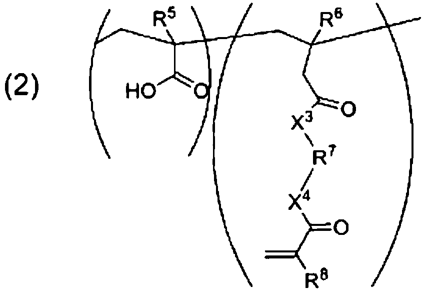

In the general formula (2), R⁵ and R⁶ each represent a hydrogen atom, a methyl group, or a carboxyl group, R⁷ represents an alkylene group having 1 to 15 carbon atoms or a polyethylene oxide segment having 2 to 7 repeating units, and R⁸ represents a hydrogen atom or a methyl group. In addition, X³ and X⁴ each represent -O- or -NH-. The number of repetitions "a" of a repeating unit on the left side and the number of repetitions "b" of a repeating unit on the right side in the general formula (2) may be identical to or different from each other. In addition, a ratio (a:b) between both the repeating units is preferably from 95:5 to 5:95, more preferably from 95:5 to 50:50, still more preferably from 90:10 to 70:30.

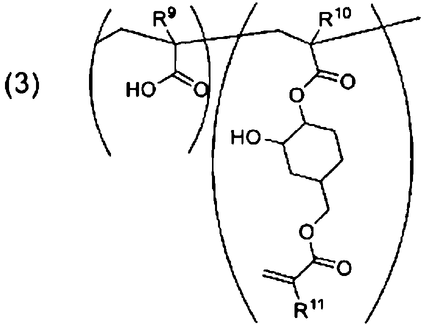

In the general formula ( 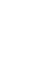 ), R⁹, R¹⁰, and R¹¹ each represent a hydrogen atom or a methyl group. The number of repetitions "a" of a repeating unit on the left side and the number of repetitions "b" of a repeating unit on the right side in the general formula (3) may be identical to or different from each other. In addition, a ratio (a:b) between both the repeating units is preferably from 95:5 to 5:95, more preferably from 80:20 to 20:80.

The weight average molecular weight (Mw) of the carboxylic acid polymer-based polymerizable monomer (a2) is from 2,000 to 120,000, preferably from 5,000 to 100,000 from the viewpoint of achieving both of an improving effect on the strength of the adhesive layer and the permeability of the carboxylic acid polymer-based polymerizable monomer (a2) into a tooth structure. The weight average molecular weight of the carboxylic acid polymer-based polymerizable monomer (a2) means a value measured by gel permeation chromatography (GPC) using polystyrene as a standard.

In addition, the acid value of the carboxylic acid polymer-based polymerizable monomer (a2) is preferably from 50 mg·KOH/g to 750 mg·KOH/g, more preferably from 100 mg·KOH/g to 600 mg·KOH/g from the viewpoint of achieving both of an improving effect on the strength of the adhesive layer and the storage stability of the one-part dental adhesive composition. The term "acid value" as used herein means the number of milligrams of potassium hydroxide (KOH) required to neutralize 1 g of a sample.

Examples of the carboxylic acid polymer-based polymerizable monomer (a2) that may be suitably used include: a polymer in which a pendant methacrylate group is introduced into a side chain of a copolymer of acrylic acid and itaconic acid, the polymer being produced by causing the copolymer of acrylic acid and itaconic acid to react with a sufficient amount of 2-isocyanatoethyl methacrylate to convert part of the acid groups of the copolymer into the pendant methacrylate group (e.g., a compound described in Example 11 of US Patent No. 5,130,347 (Mitra): VBC); a polymer (PAH20) produced by causing commercially available polyacrylic acid to react with a sufficient amount of 2-isocyanatoethyl methacrylate to convert part of the acid groups of the polyacrylic acid into a pendant methacrylate group; and Z230AA, Z250, Z254F, Z300, and Z320 of a Cyclomer-P (ACA) series (manufactured by Daicel-Allnex Ltd.). The compound corresponding to the general formula (1) is, for example, PAH20, the compound corresponding to the general formula (2) is, for example, VBC, and examples of the compound corresponding to the general formula (3) include various compounds of the Cyclomer-P (ACA) series.

In addition, from the viewpoint of improving the strength of the adhesive layer, the blending amount of the carboxylic acid polymer-based polymerizable monomer (a2) is preferably from 2 parts by mass to 40 parts by mass, more preferably from 6 parts by mass to 40 parts by mass, still more preferably from 8 parts by mass to 40 parts by mass per 100 parts by mass of the polymerizable monomers (A) included in the one-part dental adhesive composition of this embodiment.

### <<Other Acidic Group-containing Polymerizable Monomer (Other Acid Monomer) (a3)>>

The polymerizable monomers (A) may further include the other acidic group-containing polymerizable monomer (other acid monomer) (a3) except the strong acidic group-containing polymerizable monomer (a1) and the carboxylic acid polymer-based polymerizable monomer (a2) as required.

The other acidic group-containing polymerizable monomer (other acid monomer) (a3) is a polymerizable monomer having an acidic group except the strong acid monomer (a1) and the carboxylic acid polymer-based polymerizable monomer (a2). Specifically, the monomer is a polymerizable monomer, which has, in a molecule thereof, at least one radical polymerizable group and at least one acidic group (provided that a strong acidic group is excluded), and which has a weight average molecular weight of less than 2,000. The term "strong acidic group" as used herein means a strong acidic group for forming a molecule of the strong acid monomer (a1), and the term "weight average molecular weight" as used herein means a value measured by gel permeation chromatography (GPC) using polystyrene as a standard.

Examples of the radical polymerizable group include the same radical polymerizable groups as those of the strong acid monomer (a1) and the carboxylic acid polymer-based polymerizable monomer (a2). Of those, an acryloyl group and/or a methacryloyl group is preferred. In addition, the acidic group is, for example, a carboxyl group. In ordinary cases, the other acid monomer (a3) is preferably a low-molecular weight polymerizable monomer that is free of a repeating unit structure in a molecule thereof unlike a polymer, and in this case, its molecular weight generally falls within the range of from about 50 to about 800.

Examples of the other acid monomer (a3) having a carboxyl group as an acidic group may include acrylic acid, methacrylic acid, 4-(meth)acryloxyethyl trimellitic acid, 11-(meth)acryloyloxy-1,1-undecanedicarboxylic acid, 1,4-di(meth)acryloyloxy pyromellitic acid, 2-(meth)acryloyloxyethyl maleic acid, 2-(meth)acryloyloxyethyl phthalic acid, 2-(meth)acryloyloxyethyl hexahydrophthalic acid.

In addition, from the viewpoint of improving the strength of the adhesive layer, the blending amount of the other acid monomer (a3) is preferably from 0 parts by mass to 30 parts by mass, more preferably from 0 parts by mass to 15 parts by mass per 100 parts by mass of the polymerizable monomers (A) included in the one-part dental adhesive composition of this embodiment.

### 1-1-2. Acidic Group-free Polymerizable Monomer (Non-acid Monomer) (A2)

The polymerizable monomers (A) may further include the acidic group-free polymerizable monomer (non-acid monomer) (A2) as required. The acidic group-free polymerizable monomer (non-acid monomer) (A2) is a polymerizable monomer, which includes, in a molecule thereof, at least one radical polymerizable group, and which is free of an acidic group. Examples of the radical polymerizable group include the same radical polymerizable groups as those of the strong acid monomer (a1) and the carboxylic acid polymer-based polymerizable monomer (a2). Of those, an acryloyl group and/or a methacryloyl group is preferred.

Monomers that may each be suitably used as the non-acid monomer (A2) may be, for example, the following monomers. That is, examples of the non-acid monomer (A2) of a monofunctional type may include methyl (meth)acrylate, ethyl (meth)acrylate, butyl (meth)acrylate, glycidyl (meth)acrylate, 2-cyanomethyl (meth)acrylate, benzyl methacrylate, polyethylene glycol mono(meth)acrylate, allyl (meth)acrylate, 2-hydroxyethyl (meth)acrylate, 3-hydroxypropyl (meth)acrylate, and glyceryl mono(meth)acrylate.

In addition, examples of the non-acid monomer (A2) of a polyfunctional type may include 2,2'-bis{4-[2-hydroxy-3-(meth)acryloyloxypropoxy]phenyl}propane, triethylene glycol methacrylate, 2,2-bis[(4-(meth)acryloyloxypolyethoxyphenyl)propane], 1,6-bis(methacrylethyloxycarbonylamino)-2,2,4-trimethylhexane, 1,6-bis(methacrylethyloxycarbonylamino)-2,4,4-trimethylhexane, and trimethylolpropane trimethacrylate. The non-acid monomers (A2) may be used alone or in combination thereof.

In the case where the non-acid monomer (A2) is used as the polymerizable monomers (A), the content ratio of the acid monomers (A1) in the polymerizable monomers (A) is preferably from 20% by mass to 75% by mass, preferably from 30% by mass to 60% by mass. In this case, the content ratio of the non-acid monomer (A2) in the polymerizable monomers (A) corresponds to the balance excluding the acid monomers (A1) from the polymerizable monomers (A), and specifically, the content ratio is preferably from 25% by mass to 80% by mass, more preferably from 40% by mass to 70% by mass.

In addition, in the case where the strong acid monomer (a1) and the carboxylic acid polymer-based polymerizable monomer (a2), and as required, the other acid monomer (a3) and the non-acid monomer (A2) are used as the polymerizable monomers (A), the blending amount of the strong acid monomer (a1) is preferably from 3 parts by mass to 60 parts by mass, the blending amount of the carboxylic acid polymer-based polymerizable monomer (a2) is preferably from 6 parts by mass to 40 parts by mass, the blending amount of the other acid monomer (a3) is preferably from 0 parts by mass to 30 parts by mass, and the blending amount of the non-acid monomer (A2) is preferably from 25 parts by mass to 80 parts by mass per 100 parts by mass of the polymerizable monomers (A). In this case, however, the blending amounts of the above-mentioned strong acid monomer (a1), carboxylic acid polymer-based polymerizable monomer (a2), and other acid monomer (a3) are selected so that the total amount of these three components may fall within the range of from 20 parts by mass to 75 parts by mass, and at the same time, the blending amounts of the above-mentioned strong acid monomer (a1), carboxylic acid polymer-based polymerizable monomer (a2), other acid monomer (a3), and non-acid monomer (A2) are selected so that the total amount of these four components may be 100 parts by mass.

### 1-2. Photopolymerization Initiator (B)

The photopolymerization initiator (B) is a compound that initiates the radical polymerization of the polymerizable monomers (A) by light irradiation. As a result, in the one-part dental adhesive composition of this embodiment, photopolymerization type curing can be advanced. A known photopolymerization initiator may be used as the photopolymerization initiator (B) without any particular limitation. For example, α-diketones such as camphorquinone and acylphosphine oxides may each be used, and a bisacylphosphine oxide (b1) is particularly preferably used.

### <Bisacylphosphine Oxide (b1)>

The bisacylphosphine oxide (b1) is a monomolecular cleavage type highly active photopolymerization initiator. Accordingly, the bisacylphosphine oxide (b1) can advance the photopolymerization type curing of the polymerizable monomers (A) even with a small amount of light. The bisacylphosphine oxide (b1) is a compound represented by the following formula.

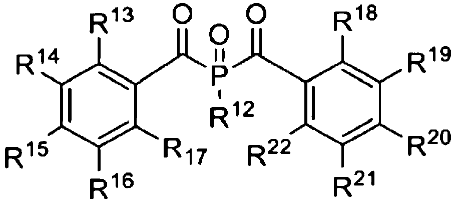

In the formula, R¹² represents an alkyl group, an alkenyl group, an alkynyl group, an aryl group, an alkoxy group, an acyl group, or an acyloxy group. R¹³ to R²² each independently represent a hydrogen atom, a halogen atom, an alkyl group, an alkenyl group, an alkynyl group, an aryl group, an alkoxy group, an acyl group, or an acyloxy group.

Examples of the bisacylphosphine oxide (b1) include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and (2,5,6-trimethylbenzoyl)-2,4,4-trimethylpentylphosphine oxide. A derivative of the bisacylphosphine oxide (b1) is also suitably used as the photopolymerization initiator (B).

In addition, from the viewpoint of obtaining high polymerization activity and high adhesive strength, the blending amount of the photopolymerization initiator (B) is preferably from 0.1 part by mass to 10 parts by mass, more preferably from 0.5 part by mass to 8 parts by mass per 100 parts by mass of the polymerizable monomers (A) included in the one-part dental adhesive composition of this embodiment.

### 1-3. Water (C)

Water that is substantially free of harmful impurities is preferably used as the water (C) from the viewpoints of the storage stability, biocompatibility, and adhesiveness of the one-part dental adhesive composition of this embodiment. For example, deionized water or distilled water may be utilized. The blending ratio of the water is preferably from 1 part by mass to 70 parts by mass, more preferably from 3 parts by mass to 60 parts by mass, still more preferably from 5 parts by mass to 50 parts by mass per 100 parts by mass of the polymerizable monomers (A) included in the one-part dental adhesive composition of this embodiment.

### 1-4. Water-soluble Organic Solvent (D)

Any known water-soluble organic solvent may be used as the water-soluble organic solvent (D) without any limitation. In ordinary cases, however, a highly volatile organic solvent having a boiling point of less than 100°C is preferably used. The blending amount of the water-soluble organic solvent (D) is preferably from 10 parts by mass to 500 parts by mass, more preferably from 30 parts by mass to 450 parts by mass per 100 parts by mass of the polymerizable monomers (A) included in the one-part dental adhesive composition of this embodiment. When the blending amount of the water-soluble organic solvent (D) is set to 10 parts by mass or more, the storage stability of the one-part dental adhesive composition can be further improved. When the blending amount is set to 500 parts by mass or less, it becomes easier to increase the thickness of an adhesive layer formed on a damaged portion of a tooth in Step 1(1), and it also becomes easier to shorten its drying time.

Specific examples of the water-soluble organic solvent (D) include: alcohols, such as methanol, ethanol, isopropyl alcohol, t-butyl alcohol, and butanol; ketones, such as acetone and methyl ethyl ketone; ethers, such as ethyl ether, 1,4-dioxane, and tetrahydrofuran; and esters, such as ethyl acetate and ethyl formate. Of those, acetone, ethanol, isopropyl alcohol, or the like is particularly preferred as the water-soluble organic solvent (D) because of, for example, the biological safety, solubility, and storage stability. Water-soluble organic solvents may be used alone or in combination thereof as the water-soluble organic solvent (D).

### 1-5. Monovalent to Tetravalent Metal Compound (E)

The monovalent to tetravalent metal compound (E) is, for example, a monovalent to tetravalent metal salt (e1) and/or a monovalent to tetravalent metal alkoxide compound (e2). The metal ion generated by dissociation from the monovalent to tetravalent metal compound (E) in the one-part dental adhesive composition after its preparation and during its storage forms an aggregate with at least part of the strong acid monomer (a1) included in the one-part dental adhesive composition. In addition, the metal ion forming the aggregate in the one-part dental adhesive composition forms an ionomer with the carboxylic acid polymer-based polymerizable monomer (a2) to form an adhesive layer having a firm ionomer structure along with the drying treatment (solvent removal from the one-part dental adhesive composition) when Step 1(1) is performed.

The metal salt (e1), for example, a hydroxide, a carboxylate such as an acetate, or a halide such as a fluoride, or the metal alkoxide compound (e2) such as an isopropoxide may be used as the monovalent to tetravalent metal compound (E). Preferred specific examples of the monovalent to tetravalent metal compound (E) that generates a monovalent metal ion, such as Li(I), Na(I), K(I), or Rb(I), may include lithium hydroxide, sodium hydroxide, sodium ethoxide, sodium fluoride, and potassium hydroxide. Preferred specific examples of the monovalent to tetravalent metal compound (E) that generates a divalent metal ion, such as Mg(II), Ca(II), Sr(II), Ba(II), Zn(II), or Cd(II), may include magnesium acetate tetrahydrate, calcium acetate monohydrate, strontium acetate hemihydrate, barium acetate, and zinc acetate dihydrate. In addition, preferred specific examples of the monovalent to tetravalent metal compound (E) that generates a trivalent metal ion, such as Sc(III), Y(III), La(III), Ce(III), Al(III), Ga(III), or In(III), may include scandium acetate hydrate, ytterbium acetate tetrahydrate, lanthanum acetate n-hydrate, cerium acetate monohydrate, aluminum triethoxide, aluminum tri-sec-butoxide, aluminum acetylacetonate, tris(ethyl acetoacetato)aluminum, and gallium acetylacetonate. Further, preferred specific examples of the monovalent to tetravalent metal compound (E) that generates a tetravalent metal ion, such as Ti(IV), Zr(IV), or Hf(IV), may include titanium tetraisopropoxide, zirconium tetrapropoxide, and zirconium tetrabutoxide.

Note that, the storage stability of the one-part dental adhesive composition and the ionomer-forming ability thereof depend on the type of metal constituting the monovalent to tetravalent metal compound (E). For example, a metal compound including a metal functioning as a catalyst, such as Cu(II) or V(IV), may impair the storage stability when blended into the one-part dental adhesive composition in an amount required to express a sufficient ionomer-forming ability. Accordingly, the metal for forming the monovalent to tetravalent metal compound (E) is preferably Na(I), Mg(II), Ti(IV), Zr(IV), Zn(II), Ga(III), or Al(III), and is more preferably Ti(IV), Ga(III), or Al(III) from the viewpoint of the adhesiveness. In addition, the monovalent to tetravalent metal compounds (E) may be used alone or in combination thereof.

In the one-part dental adhesive composition of this embodiment, <i> the content of the monovalent to tetravalent metal compound (E) per 1 mol of the strong acid monomer (a1) is from 0.02 mol to 1 mol, preferably from 0.05 mol to 0.8 mol in terms of value converted into the number of moles of the metal ion for forming the monovalent to tetravalent metal compound (E). <ii> In addition, in the one-part dental adhesive composition of this embodiment, the content of the monovalent to tetravalent metal compound (E) per 1 g of the carboxylic acid polymer-based polymerizable monomer (a2) is from 0.0002 mol to 0.005 mol, preferably from 0.0005 mol to 0.004 mol. When the content of the monovalent to tetravalent metal compound (E) satisfies the above-mentioned items <i> and <ii> simultaneously, all of such an adhesive property that the adhesive strength is unlikely to reduce even in the case where a filling material is loaded more thickly and high adhesive strength, and storage stability can be achieved. When the two or more kinds of monovalent to tetravalent metal compounds (E) are used in combination, the term "content of the monovalent to tetravalent metal compound (E)" means the total amount of values converted into the numbers of moles of the metal ions for forming the respective monovalent to tetravalent metal compounds (E) regarding the item <i>, and means the total amount of the contents of the respective monovalent to tetravalent metal compounds (E) regarding the item <ii>.

In addition, the content of the monovalent to tetravalent metal compound (E) per 100 parts by mass of the polymerizable monomers (A) is not particularly limited as long as the numerical ranges described in the above-mentioned items <i> and <ii> are satisfied. However, the content is preferably from 0.3 part by mass to 40 parts by mass, more preferably from 0.7 part by mass to 30 parts by mass.

### 1-6. Aggregate

The one-part dental adhesive composition of this embodiment includes an aggregate. The aggregate is formed by bonding the metal ion generated by dissociation from the monovalent to tetravalent metal compound (E) and at least part of the strong acid monomer (a1) included in the one-part dental adhesive composition via an ionic bond and/or a coordination bond during a process for the preparation of the one-part dental adhesive composition. The aggregate is a substance soluble in the water-soluble organic solvent (D).

The aggregate is an aggregate obtained by forming an ionic bond and/or a coordination bond between the metal ion and the strong acid monomer (a1), and hence its molecular weight is much larger than those of various low-molecular weight components included in the one-part dental adhesive composition, such as the metal ion and the strong acid monomer (a1). Accordingly, the presence of the aggregate can be easily grasped by determining its weight average molecular weight in terms of standard polystyrene measured by GPC analysis. The weight average molecular weight of the aggregate is not particularly limited, but typically falls within the range of from 1,000 to 6,000, and a range observed more frequently is from 2,000 to 5,000. For reference, in the one-part dental adhesive composition of this embodiment, the weight average molecular weight of the aggregate and the weight average molecular weight of the carboxylic acid polymer-based polymerizable monomer (a2) may overlap. However, even in such case, the molecular weight distribution of the aggregate and the molecular weight distribution of the carboxylic acid polymer-based polymerizable monomer (a2) are different from each other, and hence both the aggregate and the monomer can be easily distinguished from each other by focusing on the molecular weight distributions.

The presence of the aggregate may also be recognized by, for example, nuclear magnetic resonance (NMR) or energy dispersive X-ray (EDS) analysis utilizing a scanning electron microscope (SEM). For example, when the strong acid monomer (a1) including a phosphorus atom in a molecule thereof is used, a broad peak derived from the aggregate is observed by ³¹P NMR. This is because the broadening of the peak is expected to occur because the free rotation of bonding is inhibited by the formation of ionic crosslinking between the strong acid monomer (a1) including a phosphorus atom in a molecule thereof and the metal ion dissociated from the monovalent to tetravalent metal compound (E) in the aggregate.

In addition, in the case where solid particles are obtained by drying and solidifying a solution including the aggregate, the presence of the aggregate can be recognized by EDS analysis utilizing a SEM. In this case, when a specific atom derived from the strong acid monomer (a1) (e.g., phosphorus or sulfur for forming a strong acidic group) and a specific metal atom derived from the monovalent to tetravalent metal compound (E) (e.g., Ti or Al) can be detected simultaneously in one solid particle, which is obtained by the drying and the solidification, by the EDS analysis, it can be determined that the solid particle is a particle formed of the aggregate.

In addition, whether or not the aggregate exists in the one-part dental adhesive composition prepared after the completion of the mixing of all components can also be easily determined by visually observing whether or not insoluble matter (an ionomer formed by the aggregation of the carboxylic acid polymer-based polymerizable monomer (a2) by virtue of the metal ion dissociated from the monovalent to tetravalent metal compound (E)) is generated in the one-part dental adhesive composition. This is because of the following reason: when the insoluble matter is generated in the one-part dental adhesive composition or an intermediate composition, the metal ion required to form the aggregate has already been consumed for forming the ionomer in a system; consequently, the aggregate cannot be formed (or a sufficient amount of the aggregate is not formed).

### 1-7. Other Component

The one-part dental adhesive composition of this embodiment may include any other component except the components (A) to (E) and the aggregate as required. Examples of the other component include a filler (F), a polymerization inhibitor (G), a photopolymerization accelerator (H), a colorant, and a thickener (e.g., polymethyl (meth)acrylate). Of those, the filler (F) and/or the polymerization inhibitor (G) is preferably blended into the one-part dental adhesive composition of this embodiment. In addition, the one-part dental adhesive composition of this embodiment may be free of a chemical polymerization initiator (e.g., a redox initiator formed of a combination of an oxidizing agent and a reducing agent) because the composition is basically a composition utilized as a single-light-irradiation/photocurable type dental adhesive composition.

The filler (F) is blended to improve the mechanical strength of the adhesive layer after the curing. The filler (F) is not particularly limited, and a known inorganic filler or inorganic-organic composite filler may be used. Fumed silica is preferred as the inorganic filler. When the filler is blended, its blending amount is preferably from 5 parts by mass to 30 parts by mass, more preferably from 5 parts by mass to 25 parts by mass, still more preferably from 8 parts by mass to 20 parts by mass per 100 parts by mass of the polymerizable monomers (A) included in the one-part dental adhesive composition of this embodiment. The setting of the blending amount to 5 parts by mass or more makes it easier to obtain higher adhesive strength. The setting of the blending amount to 30 parts by mass or less secures fluidity moderate for a dental adhesive, and hence can make the operability more excellent. The fillers may be used alone or in combination thereof.

The polymerization inhibitor (G) has a function of improving the storage stability of the one-part dental adhesive composition of this embodiment. Any known polymerization inhibitor may be used as the polymerization inhibitor (G) without any limitation, and examples thereof include hydroquinone, hydroquinone monomethyl ether, and dibutylhydroxytoluene. The polymerization inhibitors (G) may be used alone or in combination thereof. The blending amount of the polymerization inhibitor (G) is preferably from 0.0001 part by mass to 10 parts by mass, more preferably from 0.001 part by mass to 5 parts by mass per 100 parts by mass of the polymerizable monomers (A) included in the one-part dental adhesive composition of this embodiment.

Any known photopolymerization accelerator may be used as the photopolymerization accelerator (H) without any limitation, but an aromatic tertiary amine compound is suitably used. Examples of the aromatic tertiary amine compound include 4-dimethylaminobenzoic acid, ethyl 4-dimethylaminobenzoate, lauryl 4-dimethylaminobenzoate, 3-dimethylaminobenzoic acid, ethyl 3-dimethylaminobenzoate, dimethylamino-p-toluidine, diethylamino-p-toluidine, and p-tolyldiethanolamine. The photopolymerization accelerators (H) may be used alone or in combination thereof. The blending amount of the photopolymerization accelerator (H) is preferably from 0.1 part by mass to 10 parts by mass, more preferably from 0.5 part by mass to 5 parts by mass per 100 parts by mass of the polymerizable monomers (A) included in the one-part dental adhesive composition of this embodiment.

### 2. Method of producing One-part Dental Adhesive Composition

The method of producing the one-part dental adhesive composition of this embodiment is not particularly limited, and for example, the one-part dental adhesive composition may be prepared by mixing all components for forming the one-part dental adhesive composition in one stroke. However, when all the components are mixed in one stroke, the monovalent to tetravalent metal compound (E), the strong acid monomer (a1), and the carboxylic acid polymer-based polymerizable monomer (a2) coexist simultaneously in a system. In such system, a reaction in which the metal ion generated by dissociation from the monovalent to tetravalent metal compound (E) associates with the strong acid monomer (a1) to form an aggregate, and a reaction in which the ion is bonded to the carboxylic acid polymer-based polymerizable monomer (a2) to form insoluble matter (an ionomer) may occur simultaneously. Accordingly, it is liable to be difficult to intentionally perform such control that only one of the reactions proceeds. Accordingly, even if the one-part dental adhesive composition is obtained by mixing all the components in one stroke, the insoluble matter (ionomer) may be included in the one-part dental adhesive composition immediately after its preparation.

Accordingly, in the method of producing the one-part dental adhesive composition of this embodiment, it is preferred that only the reaction for forming an aggregate selectively occur during a production process therefor, and that no insoluble matter (ionomer) be formed under a state immediately after the preparation of the one-part dental adhesive composition. Such production method is, for example, a production method described below.

That is, the method of producing the one-part dental adhesive composition of this embodiment produces the one-part dental adhesive composition of this embodiment that is free of any insoluble matter (ionomer) through at least: a raw material component preparation step; a first composition preparation step; and a second composition preparation step. The method of producing the one-part dental adhesive composition of this embodiment may further include any other step (e.g., a step of removing foreign matter from a solution or a third composition preparation step described later) as required in addition to the above-mentioned three steps (the raw material component preparation step, the first composition preparation step, and the second composition preparation step), or may include only the above-mentioned three steps. Details of the respective steps are described below.

### <Raw Material Component Preparation Step>

The raw material component preparation step is a step of preparing at least the strong acidic group-containing polymerizable monomer (a1), the carboxylic acid polymer-based polymerizable monomer (a2), the photopolymerization initiator (B), the water (C), the water-soluble organic solvent (D), and the monovalent to tetravalent metal compound (E) as raw material components used for the production of the one-part dental adhesive composition.

In the raw material component preparation step, a component except the components listed above may be further prepared as required. That is, the other acid monomer (a3), the non-acid monomer (A2), the filler (F), the polymerization inhibitor (G), the photopolymerization accelerator (H), or the like may be further prepared as a raw material component as required.

### <First Composition Preparation Step>

In the first composition preparation step, after the carboxylic acid polymer-based polymerizable monomer (a2) out of the respective raw material components prepared in the raw material component preparation step is excluded from options for mixing the respective raw material components with each other, at least the strong acid monomer (a1), the water-soluble organic solvent (D), and the monovalent to tetravalent metal compound (E) are selected, and the selected respective raw material components are mixed. At this time, a mixing ratio between the strong acidic group-containing polymerizable monomer (a1) and the metal ion for forming the monovalent to tetravalent metal compound (E) is set to be 1 mol:from 0.02 mol to 1 mol. The mixing ratio is preferably 1 mol:from 0.05 mol to 0.8 mol.

In the first composition preparation step, the strong acid monomer (a1) and the monovalent to tetravalent metal compound (E) are mixed under a state in which the carboxylic acid polymer-based polymerizable monomer (a2) does not exist in a system. Accordingly, the metal ion generated by dissociation from the monovalent to tetravalent metal compound (E) can be reliably bonded to the strong acid monomer (a1) to form an aggregate.

The first composition preparation step is preferably performed at a temperature near room temperature. In addition, to avoid overheating due to reaction heat generated at the time of the preparation of the first composition, the first composition may be prepared while being cooled with an ice bath as required. In the first composition preparation step, mixing and stirring are performed until a mixed liquid obtained by mixing the respective raw material components used for the preparation of the first composition becomes sufficiently homogeneous. Thus, the aggregate is sufficiently generated. Whether or not the aggregate is sufficiently generated in the first composition (whether or not an aggregate formation reaction is completely finished) can be easily recognized on the basis of whether or not insoluble matter (an ionomer) is generated when the carboxylic acid polymer-based polymerizable monomer (a2) is added to the first composition.

In addition, when the strong acid monomer (a1) is a solid monomer, or when the mixing and the stirring become difficult owing to a significant increase in viscosity in a process where the aggregate is generated after the mixing of the respective components for forming the first composition, the first composition may be prepared by further using the non-acid monomer (A2) and/or the water (C) in addition to the strong acid monomer (a1), the water-soluble organic solvent (D), and the monovalent to tetravalent metal compound (E). This is because those two components do not chemically react with the monovalent to tetravalent metal compound (E), and do not adversely affect the formation of the aggregate. Any other component except the above-mentioned two components (e.g., the filler (F)) may also be used for the preparation of the first composition as required as long as the component does not chemically react with the monovalent to tetravalent metal compound (E).

In addition, when the strong acid monomer (a1) is a solid monomer, or when the mixing and the stirring become difficult owing to a significant increase in viscosity in the process where the aggregate is generated after the mixing of the respective components for forming the first composition, the first composition may be prepared by further using the non-acid monomer (A2) and/or the water (C) in addition to the strong acid monomer (a1), the water-soluble organic solvent (D), and the monovalent to tetravalent metal compound (E). However, the addition of those two components is limited to a case in which the components do not chemically react with the monovalent to tetravalent metal compound (E), and do not adversely affect the formation of the aggregate. For example, when the monovalent to tetravalent metal compound (E) is a monovalent to tetravalent metal alkoxide compound (e2), the use of the water (C) is not preferred because the compound chemically reacts with the water (C). Meanwhile, when the monovalent to tetravalent metal compound (E) is an acetate or an acetylacetonate, the water (C) may be used because the compound does not chemically react with the water (C). Any other component except the above-mentioned two components (e.g., the filler (F)) may also be used for the preparation of the first composition as required as long as the component does not chemically react with the monovalent to tetravalent metal compound (E).

### <Second Composition Preparation Step>

In the second composition preparation step, out of the respective raw material components, which are free from being selected (for preparing the first composition) in the first composition preparation step, at least the carboxylic acid polymer-based polymerizable monomer (a2) is selected, and the selected raw material component and the first composition are mixed to prepare a second composition. At this time, a mixing ratio between the carboxylic acid polymer-based polymerizable monomer (a2) and the monovalent to tetravalent metal compound (E) is set to be 1 g:from 0.0002 mol to 0.005 mol. The mixing ratio is preferably 1 g:from 0.0005 mol to 0.004 mol.

In the second composition preparation step, at least the first composition including the aggregate and the carboxylic acid polymer-based polymerizable monomer (a2) are mixed. Then, the mixing allows the metal ion included in the aggregate and the carboxylic acid polymer-based polymerizable monomer (a2) to coexist in the system. However, the metal ion is stabilized under a state of being incorporated into the aggregate, and hence cannot react with the carboxylic acid polymer-based polymerizable monomer (a2). Accordingly, no ionomer (insoluble matter) is formed in the second composition, and hence the aggregate can stably exist also in the second composition.

In the case where all the raw material components prepared in the raw material component preparation step are selected and used up at the time of the completion of the second composition preparation step, the second composition may be used as the one-part dental adhesive composition of this embodiment. In this case, the first composition preparation step and the second composition preparation step may be performed, for example, as described below. First, in the first composition preparation step, the first composition including the aggregate is prepared by mixing the strong acid monomer (a1), the water-soluble organic solvent (D), and the monovalent to tetravalent metal compound (E). Next, in the second composition preparation step, all the remaining components (i.e., components including at least the carboxylic acid polymer-based polymerizable monomer (a2)), which are obtained by excluding the above-mentioned three components from the raw material components prepared in the raw material component preparation step, are mixed with the first composition. Thus, the second composition (in other words, the one-part dental adhesive composition of this embodiment) can be obtained.

In addition, in the case where a raw material component (residual raw material component), which is free from being selected at the time of the completion of the second composition preparation step, out of the raw material components prepared in the raw material component preparation step still exists, a third composition preparation step of preparing a third composition by mixing the second composition and the residual raw material component is performed. In this case, the third composition may be used as the one-part dental adhesive composition of this embodiment.

### Examples

The present invention is described below by way of Examples. However, the present invention is by no means limited to the following Examples.

### 1. Raw Material Component

The names and abbreviations of raw material components (provided that the water (C) and the water-soluble organic solvent (D) are excluded) used for the preparation of the one-part dental adhesive composition of each of Examples, Comparative Examples, and Reference Example are described below.

### 1-1. Polymerizable Monomers (A)

### <<Acidic Group-containing Polymerizable Monomers (Acid Monomers) (A1)>>

### <Strong Acidic Group-containing Polymerizable Monomer (Strong Acid Monomer) (a1)>

·MDP: 10-methacryloyloxydecyl dihydrogen phosphate (molecular weight: 322 g/mol)
·SPM2: mixture of mono(2-methacryloyloxyethyl) acid phosphate and bis(2-methacryloyloxyethyl) acid phosphate (molecular weight: 285 g/mol)
·PP-300P: mixture of 11-(meth)acryloyloxy tetrapropylene glycol hydrogen phosphate and 14-(meth)acryloyloxy pentapropylene glycol hydrogen phosphate (molecular weight: 427 g/mol)

### <Carboxylic Acid Polymer-based Polymerizable Monomer (a2)>

·VBC: A compound represented by the following formula in which a pendant methacrylate group was introduced into a side chain of a copolymer of acrylic acid and itaconic acid (compound synthesized in accordance with Example 11 of US Patent No. 5,130,347 (Mitra)). The characteristics of VBC are as follows: acid value: 530 mg·KOH/g (calculated value); and weight average molecular weight: from 7,000 to 8,000. The molecular weight was measured by the same method as a method described in Example 1 to be described later.

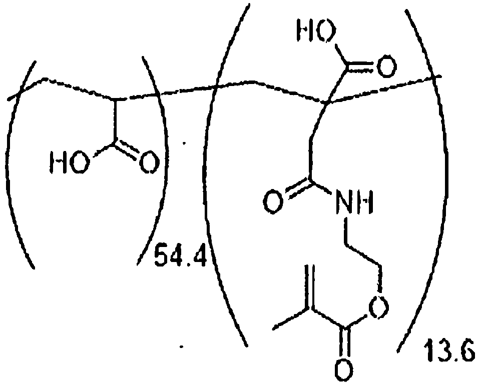

·Z230AA: acid value: from 33 mg·KOH/g to 47 mg·KOH/g, weight average molecular weight: from 10,000 to 16,000
·Z250: acid value: from 58 mg·KOH/g to 79 mg·KOH/g, weight average molecular weight: from 19,000 to 25,000
·Z254F: acid value: from 58 mg·KOH/g to 79 mg·KOH/g, weight average molecular weight: from 10,000 to 18,000
·Z300: acid value: from 102 mg·KOH/g to 144 mg·KOH/g, weight average molecular weight: from 17,000 to 25,000
·Z320: acid value: from 126 mg·KOH/g to 144 mg·KOH/g, weight average molecular weight: from 20,000 to 26,000

The above-mentioned Z230AA, Z250, Z254F, Z300, and Z320 represent the grades of a Cyclomer-P (ACA) series (manufactured by Daicel-Allnex Ltd.). In addition, a compound of any of the grades is a polymer having a main chain similar to that of an acrylic resin, and having a polymerizable group and a carboxyl group in side chains thereof. The weight average molecular weight and acid value of the compound of each grade are nominal values.
·PAH20: PAH20 was synthesized by the following procedure. First, 25 g of PAH (polyacrylic acid manufactured by FUJIFILM Wako Pure Chemical Corporation), 250 g of tetrahydrofuran (THF), 0.05 g of triphenylantimony, 0.35 g of dibutyltin dilaurate, and 11.8 g of 2-isocyanatoethyl methacrylate were weighed into a 500 mL recovery flask including a septum having placed thereinto a magnetic stirrer bar. The mixture of the above-mentioned respective components was subjected to a reaction by being stirred at 40°C for 18 hours. The resultant reaction mixture was precipitated in ethyl acetate whose volume was 20 times as large as that of the mixture. The resultant precipitate was filtered, washed with ethyl acetate, and dried in a vacuum. As a result, the final product (PAH20) was obtained with a yield of 29 g and a percentage yield of 86%. The characteristics of PAH20 are as follows: acid value: 470 mg·KOH/g; and weight average molecular weight: from 80,000 to 90,000. The molecular weight was measured by the same method as the method described in Example 1 to be described later.

### <Other Acidic Group-containing Polymerizable Monomer (Other Acid Monomer) (a3)>

·MAC-10: 11-methacryloyloxy-1,1-undecanedicarboxylic acid

### <<Acidic Group-free Polymerizable Monomer (Non-acid Monomer) (A2)>>

·BisGMA: 2,2'-bis[4-(2-hydroxy-3-methacryloyloxypropoxy)phenyl]propane
·3G: triethylene glycol dimethacrylate
·HEMA: 2-hydroxyethyl methacrylate
·UDMA: 2,2,4-trimethylhexamethylene bis(2-carbamoyloxyethyl) dimethacrylate

### 1-2. Photopolymerization Initiator (B)

·CQ: camphorquinone
·BTPO: bis(2,4,6-trimethylbenzoyl)phenyl phosphineoxide
·IC369: 2-benzyl-2-(dimethylamino)-4'-morpholinobutyrophenone (manufactured by BASF Japan Ltd., IRGACURE 369)

### 1-3. Monovalent to Tetravalent Metal Compound (E)

### <Monovalent to Tetravalent Metal Salt (e1)>

·NaOH: sodium hydroxide (molecular weight: 40 g/mol)
·Mg(OAc)₂: magnesium acetate tetrahydrate (molecular weight: 214 g/mol)
·Zn(OAc)₂: zinc acetate dihydrate (molecular weight: 220 g/mol)
·Al(acac)₃: aluminum acetylacetonate (molecular weight: 324 g/mol)
·Al(EAA)₃: tris(ethyl acetoacetato)aluminum (molecular weight: 414 g/mol)
·Ga(acac)₃: gallium acetylacetonate (molecular weight: 367 g/mol)
·Cu(OAc)₂: copper acetate monohydrate (molecular weight: 200 g/mol)

### <Monovalent to Tetravalent Metal Alkoxide Compound (e2)>

·Ti(OiPr)₄: titanium tetraisopropoxide (molecular weight: 284 g/mol)
·Zr(OPr)₄: zirconium tetrapropoxide (molecular weight: 328 g/mol)
·Al(OsBu)₃: aluminum tri-sec-butoxide (molecular weight: 246 g/mol)

### 1-4. Filler (F)

·DM-30: dimethyldichlorosilane-treated silica particles having an average primary particle diameter of 7 nm (manufactured by Tokuyama Corporation)

### 1-5. Polymerization Inhibitor (G)

·BHT: dibutylhydroxytoluene

### 1-6. Photopolymerization Accelerator (H)

·DMBE: ethyl 4-dimethylaminobenzoate

### 1-7. Other Component

·PMMA: polymethyl methacrylate (weight average molecular weight: 1,000,000)

### 2. Preparation of One-part Dental Adhesive Composition

### (Example 1)

### <First Composition Preparation Step>

A solution was prepared by mixing and stirring MDP, which was the strong acid monomer (a1), and acetone, which was the water-soluble organic solvent (D), until MDP was dissolved in acetone. Ti(OiPr)₄, which was the monovalent to tetravalent metal compound (E), was further added to the solution, and the materials were mixed and stirred for 1 hour to prepare a first composition. In the preparation, a mass ratio among MDP, acetone, and Ti(OiPr)₄ included in 292 parts by mass of the first composition was set to 35:250:7.

### <Recognition of Aggregate Formation>

The resultant first composition was subjected to weight average molecular weight measurement by GPC analysis under the following conditions through use of a diluted solution prepared by dissolving 1 part of the first composition in tetrahydrofuran (THF) whose amount was 10 times as large as that of the composition. As a result, a weight average molecular weight significantly exceeding the molecular weight of each of the raw material components used for the preparation of the first composition was measured, and its value was 3,110 in terms of standard polystyrene as shown in Table 1. Meanwhile, out of the raw material components for forming the first composition, only MDP and a Ti ion dissociated from Ti(OiPr)₄ each have a potential to be capable of forming a compound having a larger molecular weight through bonding via an ionic bond and/or a coordination bond. Accordingly, it is determined that an aggregate of MDP and the Ti ion (weight average molecular weight: 3,110) is formed in the first composition.

### [GPC Analysis Conditions]

Measurement apparatus: Advanced Polymer Chromatography (manufactured by Nihon Waters K.K.)
·Column: ACQUITY APCT^{™} XT 45, 1.7 µm
   ACQUITY APCT^{™} XT 125, 2.5 µm
·Column temperature: 40°C
·Developing solvent: THF (flow rate: 0.5 ml/min)
·Detector: photodiode array detector, 210 nm (PDA detector)

### <Second Composition Preparation Step>

Next, 292 parts by mass of the first composition was mixed with <i> 20 parts by mass of VBC, which was a carboxylic acid polymer-based polymerizable monomer, <ii> 15 parts by mass of Bis-GMA, 10 parts by mass of 3G, and 20 parts by mass of HEMA, which were non-acid monomers, <iii> 3 parts by mass of BTPO, which was a photopolymerization initiator, and <iv> 25 parts by mass of water to prepare a one-part dental adhesive composition (second composition). When the resultant one-part dental adhesive composition (second composition) was visually observed, entirely no precipitation or sedimentation of insoluble matter was observed, and hence it was found that the composition was a completely homogeneous solution. In addition, the molecular weight distribution of the second composition was recognized by performing second GPC analysis under the same conditions as those for the first composition. As a result, in the molecular weight distribution, a peak corresponding to the weight average molecular weight resulting from the aggregate, the molecular weight being measured in the first GPC analysis for the first composition, was observed. It is determined from the results of the visual observation and the second GPC analysis that an insoluble ionomer (VBC aggregated by a Ti ion) is not formed in the one-part dental adhesive composition (second composition), and that the aggregate formed in the first composition stably exists also in the second composition. The resultant one-part dental adhesive composition (second composition) was a completely homogeneous solution because entirely no precipitation or sedimentation of insoluble matter was observed even after 24 hours from its preparation as in the time of the preparation.

### (Examples 2 to 37)

One-part dental adhesive compositions of Examples 2 to 37 were each prepared by performing the first composition preparation step and the second composition preparation step in the same manner as in Example 1 on the precondition that only the strong acid monomer (a1), the water-soluble organic solvent (D), and the monovalent to tetravalent metal compound (E) were used as raw material components used for the preparation of the first composition, and except that the raw material components used for the preparation of the one-part dental adhesive composition were changed as shown in Table 2 or Table 3. In the first composition preparation step of each of Examples 2 to 7, 9, 14, 27 to 31, 35, and 36, the first composition produced in Example 1 was used instead of newly preparing a first composition. In addition, the notation "↑" in each of Table 2 and Table 3, and Table 4 to be described later means "ditto". When attention is paid to, for example, the column "Non-acid monomer (A2)" in Examples 1 to 8, the notation means that the non-acid monomers (A2) having the same composition and blending amounts as those in Example 1 are used in Examples 2 to 8.

Here, the first compositions prepared in Examples 8, 10 to 13, 15 to 26, and 32 to 34 were each subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, in each of the Examples, a measured value significantly exceeding the molecular weight of each of the raw material components was obtained, and hence it was determined that an aggregate was formed in the first composition. Table 1 shows the weight average molecular weights of the aggregates formed in the first compositions.

In addition, when the resultant one-part dental adhesive compositions (second compositions) of Examples 2 to 37 were visually observed, entirely no precipitation or sedimentation of insoluble matter was observed, and hence it was found that the compositions were completely homogeneous solutions. In addition, the molecular weight distribution of each of the second compositions was recognized by performing second GPC analysis under the same conditions as those for the first composition. As a result, in the molecular weight distribution, a peak corresponding to the weight average molecular weight resulting from the aggregate, the molecular weight being measured in the first GPC analysis for the first composition, was observed. It is determined from the results of the visual observation and the second GPC analysis that no insoluble ionomers are formed also in the one-part dental adhesive compositions (second compositions) of Examples 2 to 36, and that the aggregates formed in the first compositions stably exist also in the second compositions. In addition, the one-part dental adhesive compositions (second compositions) of Examples 2 to 37 were each a completely homogeneous solution because entirely no precipitation or sedimentation of insoluble matter was observed even after 24 hours from its preparation as in the time of the preparation.

### (Comparative Example 1)

A one-part dental adhesive composition was prepared by performing the first composition preparation step and the second composition preparation step in the same manner as in Example 1 except that: 292 parts by mass of the first composition produced in Example 1 was used as a first composition; and the raw material components used for the preparation of the one-part dental adhesive composition were changed as shown in Table 4. In Comparative Example 1, the carboxylic acid polymer-based polymerizable monomer (a2) is not used in the preparation of the one-part dental adhesive composition.

When the resultant one-part dental adhesive composition (second composition) was visually observed, entirely no precipitation or sedimentation of insoluble matter was observed, and hence it was found that the composition was a completely homogeneous solution. In Comparative Example 1, there is no room for the formation of an insoluble ionomer in the one-part dental adhesive composition (second composition) because the carboxylic acid polymer-based polymerizable monomer (a2) is not used, and for that reason, it is determined that an aggregate formed in the first composition stably exists also in the second composition.

### (Comparative Example 2)

A one-part dental adhesive composition was prepared by performing the first composition preparation step and the second composition preparation step in the same manner as in Example 1 except that the raw material components used for the preparation of the one-part dental adhesive composition were changed as shown in Table 4. In Comparative Example 2, only the strong acid monomer (a1) and the water-soluble organic solvent (D) were used as raw material components used for the preparation of the first composition, and the monovalent to tetravalent metal compound (E) was not used.

Here, the resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that no aggregate was formed in the first composition because entirely no measured value exceeding the molecular weight of each of the raw material components was obtained.

In addition, when the resultant one-part dental adhesive composition (second composition) was visually observed, entirely no precipitation or sedimentation of insoluble matter was observed, and hence it was found that the composition was a completely homogeneous solution.

### (Comparative Example 3)

A one-part dental adhesive composition was prepared by performing the first composition preparation step and the second composition preparation step in the same manner as in Example 1 except that the raw material components used for the preparation of the one-part dental adhesive composition were changed as shown in Table 4. In Comparative Example 3, only the water-soluble organic solvent (D) and the monovalent to tetravalent metal compound (E) were used as raw material components used for the preparation of the first composition, and the strong acid monomer (a1) was not used.

Here, the resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that no aggregate was formed in the first composition because entirely no measured value exceeding the molecular weight of each of the raw material components was obtained.

In addition, when the resultant one-part dental adhesive composition (second composition) was visually observed, it was recognized that a large amount of insoluble matter was generated. Accordingly, the molecular weight of a filtrate obtained by filtering and separating the insoluble matter from the second composition was measured by GPC analysis. As a result, no weight average molecular weight corresponding to the carboxylic acid polymer-based polymerizable monomer (a2) was able to be recognized for the filtrate, and hence it was found that the carboxylic acid polymer-based polymerizable monomer (a2) was not included in the filtrate. It is determined from those results that an ionomer (insoluble matter) is formed by a reaction between the metal ion dissociated from the monovalent to tetravalent metal compound (E) (that does not form any aggregate) and the carboxylic acid polymer-based polymerizable monomer (a2) during the preparation of the second composition.

### (Comparative Example 4)

A first composition was prepared by performing the first composition preparation step in the same manner as in Example 1 except that the raw material components used for the preparation of the one-part dental adhesive composition were changed as shown in Table 4. The resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that an aggregate was formed in the first composition because a measured value significantly exceeding the molecular weight of each of the raw material components was obtained. Table 1 shows the weight average molecular weight of the aggregate formed in the first composition.

Subsequently, a one-part dental adhesive composition (second composition) was prepared by performing the second composition preparation step in the same manner as in Example 1. When the resultant one-part dental adhesive composition (second composition) was visually observed, it was recognized that a large amount of insoluble matter was generated. In the composition of Comparative Example 4 shown in Table 4, an excessive amount of the monovalent to tetravalent metal compound (E) with respect to the strong acid monomer (a1) was used as compared to other Examples. Accordingly, it is conceivable that a large amount of a metal ion (excess metal ion) that was not able to participate in the formation of the aggregate was generated in the first composition, and the excess metal ion reacted with the carboxylic acid polymer-based polymerizable monomer (a2) to form an ionomer (insoluble matter) at the time of the preparation of the second composition.

### (Comparative Example 5)

A one-part dental adhesive composition (second composition) was prepared by performing the first composition preparation step and the second composition preparation step in the same manner as in Example 1 except that the raw material components used for the preparation of the one-part dental adhesive composition were changed as shown in Table 4. In Comparative Example 5, the addition amount of the monovalent to tetravalent metal compound (E) was adjusted so as to be excessive with respect to the carboxylic acid polymer-based polymerizable monomer (a2).

The resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that an aggregate was formed in the first composition because a measured value significantly exceeding the molecular weight of each of the raw material components was obtained. Table 1 shows the weight average molecular weight of the aggregate formed in the first composition.

In addition, when the resultant one-part dental adhesive composition (second composition) was visually observed, entirely no precipitation or sedimentation of insoluble matter was observed, and hence it was found that the composition was a completely homogeneous solution. In addition, the molecular weight distribution of the second composition was recognized by performing second GPC analysis under the same conditions as those for the first composition. As a result, in the molecular weight distribution, a peak corresponding to the weight average molecular weight resulting from the aggregate, the molecular weight being measured in the first GPC analysis for the first composition, was observed. It is determined from the results of the visual observation and the second GPC analysis that no insoluble ionomer is formed also in the one-part dental adhesive composition (second composition), and that the aggregate formed in the first composition stably exists also in the second composition.

### (Comparative Example 6)

In Comparative Example 6, an attempt was made to prepare a one-part dental adhesive composition in accordance with the following procedure without using the water-soluble organic solvent (D).

(i) 35 Parts by mass of MDP, which was the strong acid monomer (a1), and (ii) 15 parts by mass of Bis-GMA, 10 parts by mass of 3G, and 20 parts by mass of HEMA, which were the non-acid monomers (A2), were mixed until MDP was completely dissolved in the other monomers. Thus, a monomer mixture was obtained. Next, 9 parts by mass of Al(acac)₃, which was the monovalent to tetravalent metal compound (E), was added to the monomer mixture, and the materials were mixed for 1 hour to prepare a first composition.

Here, the resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that an aggregate was formed in the first composition because a measured value significantly exceeding the molecular weight of each of the raw material components was obtained. Table 1 shows the weight average molecular weight of the aggregate formed in the first composition.

Next, in order to prepare a one-part dental adhesive composition (second composition), 89 parts by mass of the first composition was mixed with 20 parts by mass of VBC, which was the carboxylic acid polymer-based polymerizable monomer (a2), 3 parts by mass of BTPO, which was the photopolymerization initiator (B), 25 parts by mass of the water (C), 20 parts by mass of DM-30, and 0.2 part by mass of BHT. However, the one-part dental adhesive composition (second composition) was not able to be obtained because the mixture did not include the water-soluble organic solvent (D), and hence the water and the monomers were not compatible with each other.

### (Comparative Example 7)

In Comparative Example 7, a first composition was prepared by performing the first composition preparation step in the same manner as in Example 1 except that MAC-10 containing a carboxyl group was used as the other acid monomer (a3) having lower acidity instead of the strong acid monomer (a1) during the preparation of the first composition. The resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that an aggregate was formed in the first composition because a measured value significantly exceeding the molecular weight of each of the raw material components was obtained. Table 1 shows the weight average molecular weight of the aggregate formed in the first composition.

Subsequently, a one-part dental adhesive composition (second composition) was prepared by performing the second composition preparation step in the same manner as in Example 1. When the resultant one-part dental adhesive composition (second composition) was visually observed, it was recognized that a large amount of insoluble matter was generated.

### (Comparative Example 8)

A one-part dental adhesive composition was prepared by performing the first composition preparation step and the second composition preparation step in the same manner as in Example 1 except that the raw material components used for the preparation of the one-part dental adhesive composition were changed as shown in Table 4.

Here, the resultant first composition was subjected to weight average molecular weight measurement by GPC analysis in the same manner as in Example 1, followed by the recognition of whether or not a measured value significantly exceeding the molecular weight of each of the raw material components used for the preparation of the first composition was obtained. As a result, it was determined that an aggregate was formed in the first composition because a measured value significantly exceeding the molecular weight of each of the raw material components was obtained. Table 1 shows the weight average molecular weight of the aggregate formed in the first composition.

In addition, when the resultant one-part dental adhesive composition (second composition) was visually observed, entirely no precipitation or sedimentation of insoluble matter was observed, and hence it was found that the composition was a completely homogeneous solution. In addition, the molecular weight distribution of the second composition was recognized by performing second GPC analysis under the same conditions as those for the first composition. As a result, in the molecular weight distribution, a peak corresponding to the weight average molecular weight resulting from the aggregate, the molecular weight being measured in the first GPC analysis for the first composition, was observed. It is determined from the results of the visual observation and the second GPC analysis that no insoluble ionomer is formed also in the one-part dental adhesive composition (second composition), and that the aggregate formed in the first composition stably exists also in the second composition.

### (Reference Example 1)

A related-art one-part dental adhesive composition blended with a highly active photopolymerization initiator similar to that described in Patent Literature 4 was prepared by mixing and stirring the respective raw material components shown in Table 5 in one stroke.

### 3. Evaluation of Adhesive Strength

Adhesive strength shown in each of Table 2 to Table 5 was evaluated by the following procedure. First, bovine anterior teeth extracted within 24 hours after slaughter were polished with waterproof abrasive paper P600 under water pouring, and a double-sided tape perforated with a hole having a diameter of 3 mm was pasted on a polished surface, where a dentin plane had been carved out, so as to be parallel and flat to a labial surface. Subsequently, a one-part dental adhesive composition was applied to an adhesive surface exposed from the hole of the double-sided tape in the polished surface, and was dried by air blowing for 10 seconds.

Next, paraffin wax having arranged therein a hole having a diameter of 8 mm was pasted on the adhesive surface, to which the one-part dental adhesive composition had been applied, so that the hole of the paraffin wax and the hole of the double-sided tape were concentric to each other. Thus, a simulated cavity was produced. In the production of the simulated cavity, a simulated cavity having a depth of 0.5 mm and a simulated cavity having a depth of 2.0 mm were produced by using paraffin wax having a thickness of 0.5 mm and paraffin wax having a thickness of 2.0 mm, respectively.

After that, a composite resin (Estelite Universal Flow Medium A3, manufactured by Tokuyama Dental Corporation) serving as a photocurable filling material was loaded into each of the two kinds of simulated cavities having different depths, and a polyester film was lightly brought into pressure contact with the resultant, followed by light irradiation for 10 seconds with a visible light irradiator (Elipar Deep Cure, manufactured by 3M Company, irradiation intensity: 800 mW/cm²). Thus, an adhesive layer and a filling material layer formed in the simulated cavity were simultaneously photocured. After that, a pre-polished SUS304 round bar (diameter: 8 mm, height: 18 mm) was bonded to the surface of the filling material layer after the curing with a resin cement (EsteCem II, manufactured by Tokuyama Dental Corporation). Finally, a test piece having bonded thereto the round bar was immersed in water at 37°C for 24 hours to provide a sample for adhesive strength measurement.

The tensile adhesive strength of each of the produced samples was measured with Autograph manufactured by Shimadzu Corporation (crosshead speed: 2 mm/min). The measurement of the tensile adhesive strength was performed for five samples, and the average value of the measured values of the respective samples was determined as the adhesive strength shown in each of Table 2 to Table 5. The term "CR thickness" shown in each of the tables means the thickness (mm) of the filling material layer formed in each of the simulated cavities by using the photocurable filling material (composite resin). In addition, the column "0.5" represents adhesive strength (MPa) when the CR thickness is 0.5 mm, and the column "2.0" represents adhesive strength (MPa) when the CR thickness is 2.0 mm. In addition, values shown in the column "Reduction rate" were calculated on the basis of the following equation. 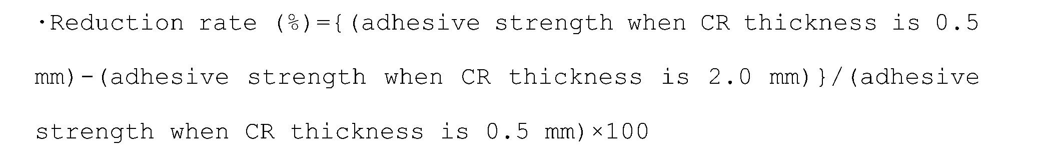 ·Reduction rate (%)={(adhesive strength when CR thickness is 0.5 mm)-(adhesive strength when CR thickness is 2.0 mm)}/(adhesive strength when CR thickness is 0.5 mm)×100

### 4. Evaluation Results

Table 1 shows the measured value of the weight average molecular weight of the first composition prepared in each of Examples, Comparative Examples, and Reference Example by the GPC analysis (provided that a measured value determined to correspond to an aggregate is shown), and the result of the visual observation of the second composition (one-part dental adhesive composition). In addition, Table 2 to Table 5 show the evaluation results of the adhesive strengths and reduction rates of the respective Examples, Comparative Examples, and Reference Example.

Numerical values shown in the columns "Components (A) to (E)" and "Other component" in each of Table 2 to Table 5 each represent parts by mass. In addition, the column "ME/Ma1" represents the content (mol) of the monovalent to tetravalent metal compound (E) per 1 mol of the strong acid monomer (a1) in terms of value converted into the number of moles of a metal ion for forming the monovalent to tetravalent metal compound (E), and the column "ME/a2" represents the content (mol) of the monovalent to tetravalent metal compound (E) per 1 g of the carboxylic acid polymer-based polymerizable monomer (a2).

**Table 1**

| | First composition | Second composition | | First composition | Second composition |
|---|---|---|---|---|---|
| | Weight average molecular weight of aggregate | Result of visual observation | | Weight average molecular weight of aggregate | Result of visual observation |
| Example 1 | 3,110 | | Example 24 | 3,162 | Homogeneous solution without precipitation or sedimentation of insoluble matter |
| Example 2 | 3,110 (*1) | | Example 25 | 2,965 | |
| Example 3 | | | Example 26 | 2,838 | |
| Example 4 | | | Example 27 | 3,110 (*1) | |
| Example 5 | | | Example 28 | | |
| Example 6 | | | Example 29 | | |
| Example 7 | | | Example 30 | | |
| Example 8 | 3,250 | | Example 31 | | |
| Example 9 | 3,110 (*1) | | Example 32 | 3,085 | |
| Example 10 | 3,080 | | Example 33 | 3,123 | |
| Example 11 | 2,952 | Homogeneous solution without precipitation or sedimentation of insoluble matter | Example 34 | 3,162 | |
| Example 12 | 2,943 | | Example 35 | 3,110 (*1) | |
| Example 13 | 3,329 | | Example 36 | | |
| Example 14 | 3,110 (*1) | | Example 37 | 4,250 (*2) | |
| Example 15 | 2,963 | | Comparative Example 1 | 3,110 (*1) | Homogeneous solution without precipitation or sedimentation of insoluble matter |
| Example 16 | 4,300 | | Comparative Example 2 | No aggregate was formed | |
| Example 17 | 4,163 | | Comparative Example 3 | No aggregate was formed | A large amount of insoluble matter was generated |
| Example 18 | 4,250 | | Comparative Example 4 | 4,322 | A large amount of insoluble matter was generated |
| Example 19 | 4,120 | | Comparative Example 5 | 4,215 | Homogeneous solution without precipitation or sedimentation of insoluble matter |
| Example 20 | 3,914 | | Comparative Example 6 | 3,058 | Visual observation was impossible (second composition was not able to be prepared) |
| Example 21 | 2,824 | | Comparative Example 7 | 2,803 | A large amount of insoluble matter was generated |
| Example 22 | 3,823 | | Comparative Example 8 | 4,977 | Homogeneous solution without precipitation or sedimentation of insoluble matter |
| Example 23 | 3,250 | | | | |

| | | | | | |
|---|---|---|---|---|---|
| *1: The first composition used in each of Examples 2 to 7, 9, 14, 27 to 31, 35, and 36, and Comparative Example 1 is the same as the first composition prepared in Example 1. *2: The first composition used in Example 37 is the same as the first composition prepared in Example 18. | | | | | |

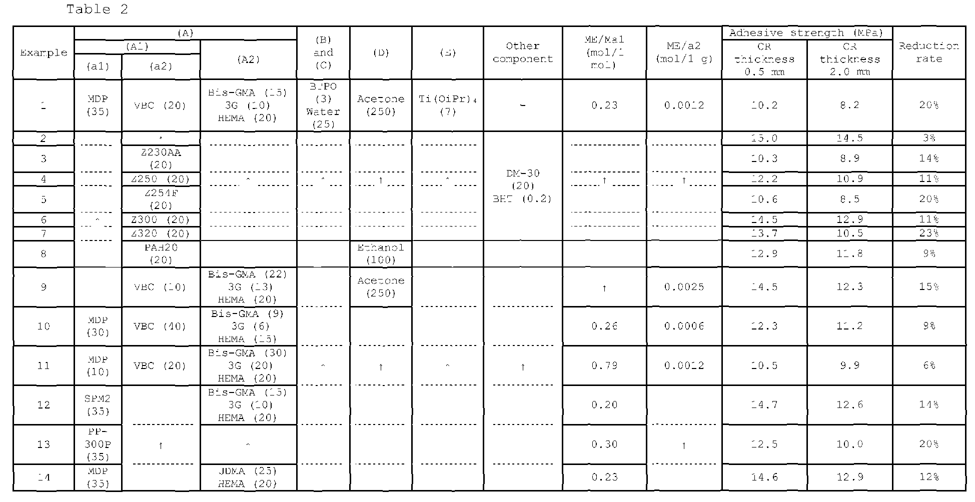

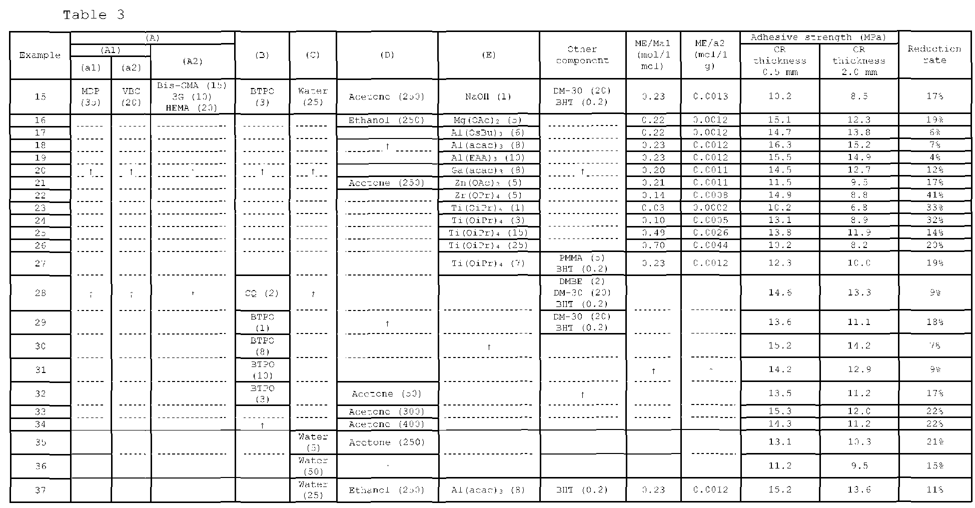

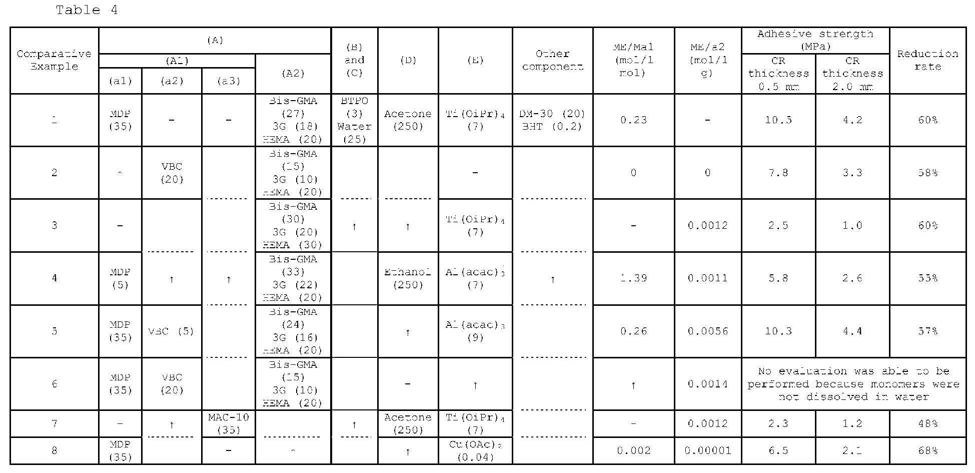

**Table 5**

| Reference Example | (A) | | | (B) and (C) | (D) | Other component | Adhesive strength (MPa) | | Reduction rate |
|---|---|---|---|---|---|---|---|---|---|
| | (A1) | | (A2) | | | | CR thickness 0.5 mm | CR thickness 2.0 mm | |
| | (a1) | (a2) | | | | | | | |
| 1 | MDP (35) | - | Bis-GMA (27) | IC369 (3) | Acetone (250) | DM-30 (20) | 15.2 | 2.3 | 85% |
| | | | 3G (18) | Water (25) | | BHT (0.2) | | | |
| | | | HEMA (20) | | | | | | |

As shown in Table 2 and Table 3, in each of Examples, the adhesive strength tended to be unlikely to reduce even when the CR thickness increased from 0.5 mm to 2.0 mm, and hence it was found that relatively high adhesive strength was obtained even when the CR thickness was 2.0 mm.

Meanwhile, as shown in Table 4, in Comparative Example 1, a reduction in adhesive strength along with an increase in CR thickness was significant because the carboxylic acid polymer-based polymerizable monomer (a2) was not included. In Comparative Example 2, a reduction in adhesive strength along with an increase in CR thickness was significant because the monovalent to tetravalent metal compound (E) was not included.

In Comparative Example 3, the component (strong acid monomer (a1)) for stabilizing the metal ion dissociated from the monovalent to tetravalent metal compound (E) as an aggregate in the one-part dental adhesive composition was not used. Accordingly, no aggregate was formed in the resultant one-part dental adhesive composition, and a large amount of the insoluble matter (ionomer) was generated. As a result, the adhesive strength was remarkably low, and the reduction rate was large. In Comparative Example 4, the ratio "ME/Ma1" was set to exceed 1 mol/1 mol. Accordingly, a large amount of the insoluble matter (ionomer) was generated in the resultant one-part dental adhesive composition. As a result, the adhesive strength was low, and the reduction rate was large.

In Comparative Example 5, the ratio "ME/a2" was set to exceed 0.005 mol/g. Accordingly, a reduction in adhesive strength along with an increase in CR thickness was significant. In Comparative Example 6, no one-part dental adhesive composition was able to be prepared because the water-soluble organic solvent (D) was not used. In Comparative Example 7, the one-part dental adhesive composition was prepared by using the carboxyl group-containing polymerizable monomer as the other acid monomer (a3) instead of the strong acid monomer (a1), but the insoluble matter was generated in the one-part dental adhesive composition. As a result, the adhesive strength was remarkably low, and the reduction rate was large. In Comparative Example 8, the formation of the aggregate was recognized by the GPC analysis, and no insoluble matter was observed in the one-part dental adhesive composition. However, a reduction in adhesive strength along with an increase in CR thickness was significant because the values of the ratios "ME/Ma1" and "ME/a2" were too small.

In addition, in Reference Example 1, the related-art one-part dental adhesive composition blended with the highly active photopolymerization initiator described in Patent Literature 4 was evaluated for its adhesive strength. Although the composition showed excellent adhesive strength when the CR thickness was 0.5 mm, its adhesive strength remarkably reduced when the CR thickness was 2.0 mm.

## Claims

1. A one-part dental adhesive composition, which is used for bonding a photocurable filling material to a tooth structure, the composition comprising:
polymerizable monomers (A) including acidic group-containing polymerizable monomers (A1);
a photopolymerization initiator (B);
water (C);
a water-soluble organic solvent (D); and
a monovalent to tetravalent metal compound (E),
wherein the acidic group-containing polymerizable monomers (A1) include:
a strong acidic group-containing polymerizable monomer (a1) having, in a molecule thereof, at least one radical polymerizable group and at least one strong acidic group selected from the group consisting of: a phosphono group {-P(=O)(OH)₂}; a dihydrogen phosphate monoester group {-O-P(=O)(OH)₂}; a hydrogen phosphate diester group {(-O-)₂P(=O)OH}; and a sulfo group (-SO₃H); and
a carboxylic acid polymer-based polymerizable monomer (a2), which has, in a molecule thereof, at least one radical polymerizable group and at least one carboxyl group, which is free of the strong acidic group, and which has a weight average molecular weight (Mw) of from 2,000 to 120,000,
wherein a content of the monovalent to tetravalent metal compound (E) per 1 mol of the strong acidic group-containing polymerizable monomer (a1) is from 0.02 mol to 1 mol in terms of value converted into the number of moles of a metal ion for forming the monovalent to tetravalent metal compound (E),
wherein a content of the monovalent to tetravalent metal compound (E) per 1 g of the carboxylic acid polymer-based polymerizable monomer (a2) is from 0.0002 mol to 0.005 mol, and
wherein the metal ion for forming the monovalent to tetravalent metal compound (E) forms an aggregate with at least part of the strong acidic group-containing polymerizable monomer (a1).

2. The one-part dental adhesive composition according to claim 1, wherein the one-part dental adhesive composition comprises 6 parts by mass to 60 parts by mass of the strong acidic group-containing polymerizable monomer (a1) per 100 parts by mass of the polymerizable monomers (A).

3. The one-part dental adhesive composition according to claim 1 or 2, wherein the one-part dental adhesive composition comprises 6 parts by mass to 40 parts by mass of the carboxylic acid polymer-based polymerizable monomer (a2) per 100 parts by mass of the polymerizable monomers (A).

4. The one-part dental adhesive composition according to claim 1 or 2, wherein a weight average molecular weight in terms of standard polystyrene measured by gel permeation chromatography analysis of the aggregate is from 1,000 to 6,000.

5. The one-part dental adhesive composition according to claim 1 or 2, wherein an acid value of the carboxylic acid polymer-based polymerizable monomer (a2) is from 50 mg·KOH/g to 750 mg·KOH/g.

6. The one-part dental adhesive composition according to claim 1 or 2, wherein the one-part dental adhesive composition comprises 10 parts by mass to 500 parts by mass of the water-soluble organic solvent (D) per 100 parts by mass of the polymerizable monomers (A).

7. The one-part dental adhesive composition according to claim 1 or 2, further comprising 5 parts by mass to 30 parts by mass of a filler (F) per 100 parts by mass of the polymerizable monomers (A).

8. The one-part dental adhesive composition according to claim 1 or 2, wherein the monovalent to tetravalent metal compound (E) includes at least one kind of metal selected from the group consisting of: Na(I); Mg(II); Ti(IV); Zr(IV); Zn(II); Ga(III); and Al(III).

9. A method of producing a one-part dental adhesive composition, the method comprising at least:
a raw material component preparation step of preparing at least a strong acidic group-containing polymerizable monomer (a1), a carboxylic acid polymer-based polymerizable monomer (a2), a photopolymerization initiator (B), water (C), a water-soluble organic solvent (D), and a monovalent to tetravalent metal compound (E) as raw material components;
a first composition preparation step of preparing a first composition by selecting at least the strong acidic group-containing polymerizable monomer (a1), the water-soluble organic solvent (D), and the monovalent to tetravalent metal compound (E) after excluding, out of the raw material components prepared in the raw material component preparation step, the carboxylic acid polymer-based polymerizable monomer (a2) from options for mixing the raw material components with each other, and mixing the selected raw material components; and
a second composition preparation step of preparing a second composition by selecting at least the carboxylic acid polymer-based polymerizable monomer (a2) from the raw material components, which are free from being selected in the first composition preparation step, and mixing the selected raw material component and the first composition, to produce the one-part dental adhesive composition of claim 1 or 2,
wherein in the first composition preparation step, a mixing ratio between the strong acidic group-containing polymerizable monomer (a1) and a metal ion for forming the monovalent to tetravalent metal compound (E) is set to be 1 mol:from 0.02 mol to 1 mol, and
wherein in the second composition preparation step, a mixing ratio between the carboxylic acid polymer-based polymerizable monomer (a2) and the monovalent to tetravalent metal compound (E) is set to be 1 g:from 0.0002 mol to 0.005 mol.
